# FASCICULE DE BREVET EUROPEEN

(11) **EP 1 720 848 B1**
(45) Date de publication et mention de la délivrance du brevet: **05.12.2007**
(21) Numéro de dépôt: 05732897.3
(22) Date de dépôt: 25.02.2005
(51) Int. Cl.: C07D 295/185, A61K 31/496, C07D 261/08, C07D 209/16, A61K 31/495, C07D 231/12, C07D 239/26, A61K 31/506, C07D 277/28, A61P 29/00, A61P 25/00, A61P 35/00, A61P 37/00, A61P 31/00, A61P 33/00

(54) **DERIVES DE ALKYLPIPERAZINE- ET ALKYLHOMOPIPERAZINE- CARBOXYLATES, LEUR PREPARATION ET LEUR APPLICATION EN TANT QU'INHIBITEURS DE L'ENZYME FAAH**
DERIVATE VON ALKYLPIPERAZIN- UND ALKYLHOMOPIPERAZINCARBOXYLATEN, VERFAHREN ZU DEREN HERSTELLUNG UND DEREN VERWENDUNG ALS INHIBITOREN DES FAAH-ENZYMS
DERIVATIVES OF ALKYLPIPERAZINE- AND ALKYLHOMOPIPERAZINE- CARBOXYLATES, PREPARATION METHOD THEREOF AND USE OF SAME AS FAAH ENZYME INHIBITORS

(30) Priorité: 26.02.2004 FR 0401953
(43) Date de publication de la demande: 15.11.2006
(73) Titulaire: Sanofi-Aventis, 75013 Paris (FR)
(72) Inventeur: ABOUABDELLAH, Ahmed, F-94320 Thiais (FR); ALMARIO GARCIA, Antonio, F-92290 Chatenay Malabry (FR); HOORNAERT, Christian, F-92160 Antony (FR); LI, Adrien, Tak, F-92260 Fontenay aux Roses (FR)
(74) Mandataire: Morel-Pécheux, Muriel
(86) Numéro de dépôt international: PCT/FR2005/000450
(87) Numéro de publication internationale: WO 2005/090322

(56) Documents cités:
- WO-A-01/72728
- WO-A-97/14689
- WO-A-03/097573
- WO-A-20/04099176
- WO-A-20/05070910
- DE-A- 19 816 889

## Description

L'invention a pour objet des dérivés de alkylpipérazine- et alkylhomopipérazine-carboxylates, leur préparation et leur application en thérapeutique.

On connaît déjà des dérivés de phénylalkylcarbamates, de dioxane-2-alkylcarbamates et de 1-pipérazine- et 1-homopipérazine-carboxylates, décrits respectivement dans les documents WO2004/067498 A, WO2004/020430 A et WO2005/070910 A, inhibiteurs de l'enzyme FAAH (Fatty Acid Amido Hydrolase).
Il existe toujours une nécessité de trouver et de développer des produits inhibiteurs de l'enzyme FAAH. Les composés de l'invention répondent à ce but.

Les composés de l'invention répondent à la formule générale (I) 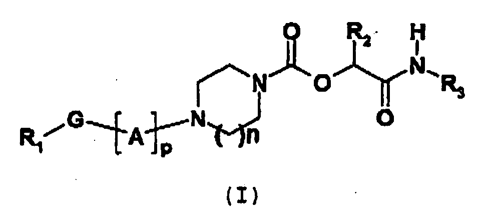 dans laquelle
n représente un nombre entier égal à 1 ou 2 ;
p représente un nombre entier allant de 1 à 7 ;
A est choisi parmi un ou plusieurs groupes X, Y et/ou Z ;
X représente un groupe méthylène éventuellement substitué par un ou deux groupes C₁₋₆-alkyle, C₃₋₇-cycloalkyle ou C₃₋₇-cycloalkyl-C₁₋₃-alkylène;
Y représente soit un groupe C₂-alcènylène éventuellement substitué par un ou deux groupes C₁₋₆-alkyle, C₃₋₇-cycloalkyle ou C₃₋₇-cycloalkyl-C₁₋₃-alkylène; soit un groupe C₂-alcynylène ;
Z représente un groupe de formule : 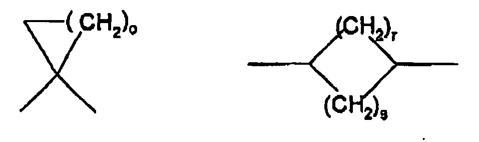
o représente un nombre entier allant de 1 à 5 ;
r et s représentent des nombres entiers et sont définis tels que r+s soit un nombre allant de 1 à 5 ;
G représente une liaison simple, un atome d'oxygène ou de soufre, un groupe SO, SO₂, C=O ou CH (OH) ;
R₁ représente un groupe R₄ éventuellement substitué par un ou plusieurs groupes R₅ et/ou R₆ ;
R₄ représente un groupe choisi parmi un phényle, pyridinyle, pyrimidinyle, pyridazinyle, pyrazinyle, triazinyle, pyrrolyle, furanyle, thiènyle, imidazolyle, oxazolyle, thiazolyle, pyrazolyle, isoxazolyle, isothiazolyle, oxadiazolyle, thiadiazolyle, triazolyle, tétrazolyle, naphtalènyle, diphénylméthyle, quinolinyle, tétrahydroquinolinyle, isoquinolinyle, tétrahydroisoquinolinyle, quinazolinyle, quinoxalinyle, phthalazinyle, cinnolinyle, naphthyridinyle, benzofuranyle, dihydrobenzofuranyle, benzothiènyle, dihydrobenzothiènyle, indolyle, indolinyle, indanyle, indazolyle, isoindolyle, benzimidazolyle, benzoxazolyle, benzisoxazolyle, benzothiazolyle, benzisothiazolyle, benzotriazolyle, benzoxadiazolyle, benzothiadiazolyle, pyrrolopyridinyle, furopyridinyle, thiènopyridinyle, imidazopyridinyle, oxazolopyridinyle, thiazolopyridinyle, pyrazolopyridinyle, isoxazolopyridinyle, isothiazolopyridinyle ;
R₅ représente un atome d'halogène ou un groupe cyano, nitro, C₁₋₆-alkyle, C₁₋₆-alcoxy, hydroxyle, C₁₋₆-thioalkyle, C₁₋₆-fluoroalkyle, C₁₋₆-fluoroalcoxy, C₁₋₆-fluorothioalkyle, NR₇R₈, NR₇COR₈, NR₇CO₂R₈, NR₇SO₂R₈, COR₇, CO₂R₇, CONR₇R₈, SO₂R₇, SO₂NR₇R₈ ou -O- (C₁₋₃-alkylène) -O ;
R₆ représente un groupe phényle, phényloxy, benzyloxy, naphtalènyle, pyridinyle, pyrimidinyle, pyridazinyle ou pyrazinyle ; le ou les groupes R₆ étant éventuellement substitués par un ou plusieurs groupes R₅ identiques ou différents l'un de l'autre ;
R₇ et R₈ représentent indépendamment l'un de l'autre un atome d'hydrogène ou un groupe C₁₋₆-alkyle, ou forment avec le ou les atomes qui les portent un cycle choisi parmi azétidine, pyrrolidine, pipéridine, morpholine, thiomorpholine, azépine, pipérazine, ce cycle étant éventuellement substitué par un groupe C₁₋₆-alkyle ou benzyle ;
R₂ représente un atome d'hydrogène ou un groupe C₁₋₆-alkyle ;
R₃ représente un atome d'hydrogène ou un groupe C₁₋₆-alkyle, C₃₋₇-cycloalkyle, C₃₋₇-cycloalkyl-C₁₋₃-alkyle.

Dans le cadre de l'invention, les composés de formule générale (I) peuvent donc comporter plusieurs groupes A identiques ou différents entre eux.

Parmi les composés de formule générale (I), un premier sous-groupe de composés est constitué des composés pour lesquels :
n représente un nombre entier égal à 1 ou 2 ;
p représente un nombre entier allant de 1 à 7 ;
A est choisi parmi un ou plusieurs groupes X et/ou Y ;
X représente un groupe méthylène éventuellement substitué par un ou deux groupes C₁₋₆-alkyle, plus particulièrement méthyle;
Y représente soit un groupe C₂-alcènylène, soit un groupe C₂-alcynylène ;
G représente une liaison simple, un atome d'oxygène ou un groupe C=O ;
R₁ représente un groupe R₄ éventuellement substitué par un ou plusieurs groupes R₅ et/ou R₆ ;
R₄ représente un groupe choisi parmi un phényle, naphtalènyle, diphénylméthyle, quinolinyle, indolyle, pyrazolyle, isoxazolyle, pyrimidinyle, thiazolyle ;
R₅ représente un atome d'halogène, plus particulièrement un chlore, un fluor, un brome ou un iode, ou un groupe cyano, C₁₋₆-alkyle, plus particulièrement un méthyle, un isopropyle ou un *tert*-butyle, C₁₋₆-alcoxy, plus particulièrement un méthoxy, C₁₋₆-fluoroalkyle, plus particulièrement un trifluorométhyle, C₁₋₆-fluoroalcoxy, plus particulièrement un trifluorométhoxy, ou -O-(C₁₋₃-alkylène)-O, plus particulièrement un -OCH₂O- ;
R₆ représente un groupe phényle, naphtalènyle ou benzyloxy ;
R₂ représente un atome d'hydrogène ou un groupe C₁₋₆-alkyle ;
R₃ représente un atome d'hydrogène ou un groupe C₁₋₆-alkyle, C₃₋₇-cycloalkyle, C₃₋₇-cycloalkyl-Cl₁₋₃-alkyle.

Parmi les composés de formule générale (I), un second sous-groupe de composés est constitué des composés pour lesquels :
n représente un nombre entier égal à 1 ;
p représente un nombre entier allant de 1 à 4 ;
A est choisi parmi un ou plusieurs groupes X et/ou Y ;
X représente un groupe méthylène éventuellement substitué par un ou deux groupes C₁₋₆-alkyle, plus particulièrement méthyle;
Y représente un groupe C₂-alcynylène ;
G représente une liaison simple ou un atome d'oxygène ;
R₁ représente un groupe R₄ éventuellement substitué par un ou plusieurs groupes R₅ et/ou R₆ ;
R₄ représente un groupe choisi parmi un phényle, naphtalènyle, isoxazolyle ;
R₅ représente un atome d'halogène, plus particulièrement un chlore ou un fluor, ou un groupe cyano, C₁₋₆-alcoxy, plus particulièrement un méthoxy, C₁₋₆-fluoroalkyle, plus particulièrement un trifluorométhyle;
R₆ représente un groupe phényle ;
R₂ représente un atome d'hydrogène ou un groupe C₁₋₆-alkyle;
R₃ représente un atome d'hydrogène ou un groupe C₁₋₆-alkyle, C₃₋₇-cycloalkyle, C₃₋₇-cycloalkyl-C₁₋₃-alkyle.

Parmi les composés de formule générale (I), un troisième sous-groupe de composés est constitué des composés pour lesquels :
n, p, A, X, Y, Z, o, r, s, G, R₁, R₄, R₅, R₆, R₇ et R₈ sont tels que définis dans la formule générale (I) ou dans les sous-groupes tels que définis ci-dessus ;
R₂ représente un atome d'hydrogène ;
R₃ représente un atome d'hydrogène ou un groupe C₁₋₆-alkyle, plus particulièrement un méthyle, C₃₋₇-cycloalkyle, plus particulièrement un cyclopropyle, ou C₃₋₇-cycloalkyl-C₁₋₃-alkyle, plus particulièrement un -CH₂-cyclopropyle.

Parmi les composés de formule générale (I), les composés suivants peuvent être cités :
- 4-(2-biphényl-3-yléthyl)pipérazine-1-carboxylate de 2-(méthylamino)-2-oxoéthyle
- 4-(2-biphényl-4-yléthyl)pipérazine-1-carboxylate de 2-(méthylamino)-2-oxoéthyle
- 4-[2-(1-naphtyl)éthyl]pipérazine-1-carboxylate de 2-(méthylamino)-2-oxoéthyle
- 4-{2-[3-(4-chlorophényl)isoxazol-5-yl]éthyl}pipérazine-1-carboxylate de 2-(méthylamino)-2-oxoéthyle
- 4-{2-[5-(4-chlorophényl)isoxazol-3-yl]éthyl}pipérazine-1-carboxylate de 2-(méthylamino)-2-oxoéthyle
- 4-(3-biphényl-3-ylpropyl)pipérazine-1-carboxylate de 2-(méthylamino)-2-oxoéthyle
- 4-(3-biphényl-4-ylpropyl)pipérazine-1-carboxylate de 2-(méthylamino)-2-oxoéthyle
- 4-(3-biphényl-3-yl-1,1-diméthylpropyl)pipérazine-1-carboxylate de 2-(méthylamino)-2-oxoéthyle
- 4-[3-(3'-chlorobiphényl-3-yl)propyl]pipérazine-1-carboxylate de 2-(méthylamino)-2-oxoéthyle
- 4-[3-(4'-chlorobiphényl-3-yl)propyl]pipérazine-1-carboxylate de 2-(méthylamino)-2-oxoéthyle
- 4-[3-(3'-methoxybiphényl-3-yl)propyl]pipérazine-1-carboxylate de 2-(méthylamino)-2-oxoéthyle
- 4-[3-(4'-methoxybiphényl-3-yl)propyl]pipérazine-1-carboxylate de 2-(méthylamino)-2-oxoéthyle
- 4-[3-(3'-chlorobiphényl-4-yl)propyl]pipérazine-1-carboxylate de 2-(méthylamino)-2-oxoéthyle
- 4-[3-(4'-chlorobiphényl-4-yl)propyl]pipérazine-1-carboxylate de 2-(méthylamino)-2-oxoéthyle
- 4-[3-(2-naphtyl)propyl]pipérazine-1-carboxylate de 2-(méthylamino)-2-oxoéthyle
- 4-{3-[5-(4-chlorophényl)isoxazol-3-yl]propyl}pipérazine-1-carboxylate de 2-(méthylamino)-2-oxoéthyle
- 4-{3-[3-(4-chlorophényl)isoxazol-5-yl]propyl}pipérazine-1-carboxylate de 2-(méthylamino)-2-oxoéthyle
- 4-[4-(3-chlorophényl)butyl]pipérazine-1-carboxylate de 2-(méthylamino)-2-oxoéthyle
- 4-[4-(4-chlorophényl)butyl]pipérazine-1-carboxylate de 2-(méthylamino)-2-oxoéthyle
- 4-{4-[3-(trifluorométhyl)phényl]butyl}pipérazine-1-carboxylate de 2-(méthylamino)-2-oxoéthyle
- 4-{4-[4-(trifluorométhyl)phényl]butyl}pipérazine-1-carboxylate de 2-(méthylamino)-2-oxoéthyle
- 4-{4-[4- (trifluorométhyl)phényl]but-3-yn-1-yl}pipérazine-1-carboxylate de 2-(méthylamino)-2-oxoéthyle
- 4-[5-(3-chlorophényl)pent-4-yn-1-yl]pipérazine-1-carboxylate de 2-(méthylamino)-2-oxoéthyle
- 4-[5-(2,4-dichlorophényl)pent-4-yn-1-yl]pipérazine-1-carboxylate de 2-(méthylamino)-2-oxoéthyle
- 4-[5-(2,5-dichlorophényl)pent-4-yn-1-yl]pipérazine-1-carboxylate de 2-(méthylamino)-2-oxoéthyle
- 4-[5-(3,4-dichlorophényl)pent-4-yn-1-yl]pipérazine-1-carboxylate de 2-(méthylamino)-2-oxoéthyle
- 4-[5-(3-chloro-4-fluorophényl)pent-4-yn-1-yl]pipérazine-1-carboxylate de 2-(méthylamino)-2-oxoéthyle
- 4-[3-(2-chlorophénoxy)propyl]pipérazine-1-carboxylate de 2-(méthylamino)-2-oxoéthyle
- 4-[3-(3-chlorophénoxy)propyl]pipérazine-l-carboxylate de 2-(méthylamino)-2-oxoéthyle
- 4-[3-(4-chlorophénoxy)propyl]pipérazine-1-carboxylate de 2-(méthylamino)-2-oxoéthyle
- 4-[3-(2,3-dichlorophénoxy)propyl]pipérazine-1-carboxylate de 2-(méthylamino)-2-oxoéthyle
- 4-[3-(2,4-dichlorophénoxy)propyl]pipérazine-1-carboxylate de 2-(méthylamino)-2-oxoéthyle
- 4-[3-(2,5-dichlorophénoxy)propyl]pipérazine-1-carboxylate de 2-(méthylamino)-2-oxoéthyle
- 4-[3-(2,6-dichlorophénoxy)propyl]pipérazine-1-carboxylate de 2-(méthylamino)-2-oxoéthyle
- 4-[3-(3,5-dichlorophénoxy)propyl]pipérazine-1-carboxylate de 2-(méthylamino)-2-oxoéthyle.

Les composés de formule générale (I) peuvent comporter un ou plusieurs carbones asymétriques. Ils peuvent exister sous forme d'énantiomères ou de diastéréoisomères. Les composés de formule générale (I) peuvent également exister sous forme de stéréoisomères cis (Z) ou trans (E). Ces stéréoisomères, énantiomères et diastéréoisomères, ainsi que leurs mélanges, y compris les mélanges racémiques, font partie de l'invention.

Les composés de formule (I) peuvent exister à l'état de bases ou de sels d'addition à des acides. De tels sels d'addition font partie de l'invention.

Ces sels sont avantageusement préparés avec des acides pharmaceutiquement acceptables, mais les sels d'autres acides utiles, par exemple, pour la purification ou l'isolement des composés de formule (I) font également partie de l'invention.

Les composés de formule générale (I) peuvent se trouver sous forme d'hydrates ou de solvats, à savoir sous forme d'associations ou de combinaisons avec une ou plusieurs molécules d'eau ou avec un solvant. De tels hydrates et solvats font également partie de l'invention.

Dans le cadre de l'invention, on entend par :
- C_{t-z} où t et z peuvent prendre les valeurs de 1 à 7, une chaîne carbonée pouvant avoir de t à z atomes de carbone, par exemple C₁₋₃ une chaîne carbonée qui peut avoir de 1 à 3 atomes de carbone ;
- alkyle, un groupe aliphatique saturé, linéaire ou ramifié; par exemple un groupe C₁₋₆-alkyle représente une chaîne carbonée de 1 à 6 atomes de carbone, linéaire ou ramifiée, plus particulièrement un méthyle, éthyle, propyle, isopropyle, butyle, isobutyle, tert-butyle, pentyle, hexyle ;
- alkylène, un groupe alkyle divalent saturé, linéaire ou ramifié, par exemple un groupe C₁₋₃-alkylène représente une chaîne carbonée divalente de 1 à 3 atomes de carbone, linéaire ou ramifiée, plus particulièrement un méthylène, éthylène, 1-méthyléthylène, propylène ;
- cycloalkyle, un groupe alkyle cyclique, par exemple un groupe C₃₋₇-cycloalkyle représente un groupe carboné cyclique de 3 à 7 atomes de carbone, plus particulièrement un cyclopropyle, cyclobutyle, cyclopentyle, cyclohexyle, cycloheptyle ;
- alcènylène, un groupe aliphatique à 2 carbones, insaturé divalent, plus particulièrement un éthylène,
- C₂-alcynylène, un groupe -C≡C- ;
- alcoxy, un groupe -O-alkyle à chaîne aliphatique saturée, linéaire ou ramifiée ;
- thioalkyle, un groupe -S-alkyle à chaîne aliphatique saturée, linéaire ou ramifiée ;
- fluoroalkyle, un groupe alkyle dont un ou plusieurs atomes d'hydrogène ont été substitués par un atome de fluor ;
- fluoroalcoxy, un groupe alcoxy dont un ou plusieurs atomes d'hydrogène ont été substitués par un atome de fluor ;
- fluorothioalkyle, un groupe thioalkyle dont un ou plusieurs atomes d'hydrogène ont été substitués par un atome de fluor ;
- atome d'halogène, un fluor, un chlore, un brome ou un iode.

Les composés de l'invention peuvent être préparés selon différentes méthodes, illustrées par les schémas qui suivent.

Ainsi selon une première méthode (schéma 1), les composés de formule générale (I) peuvent être préparés en faisant réagir une amine de formule générale (IV), dans laquelle R₁, G, A, p et n sont tels que définis dans la formule générale (I), avec un carbonate de formule générale (IIIa) dans laquelle V représente un atome d'hydrogène ou un groupe nitro, R₂ est tel que défini dans la formule générale (I) et R représente un groupe méthyle ou éthyle. Le carbamate-ester de formule générale (II) ainsi obtenu est ensuite transformé en composé de formule générale (I), par aminolyse au moyen d'une amine de formule générale R₃NH₂ dans laquelle R₃ est tel que défini dans la formule générale (I). La réaction d'aminolyse peut être réalisée dans un solvant tel que le méthanol ou l'éthanol, ou dans un mélange de solvants tels que le méthanol et le tétrahydrofurane.

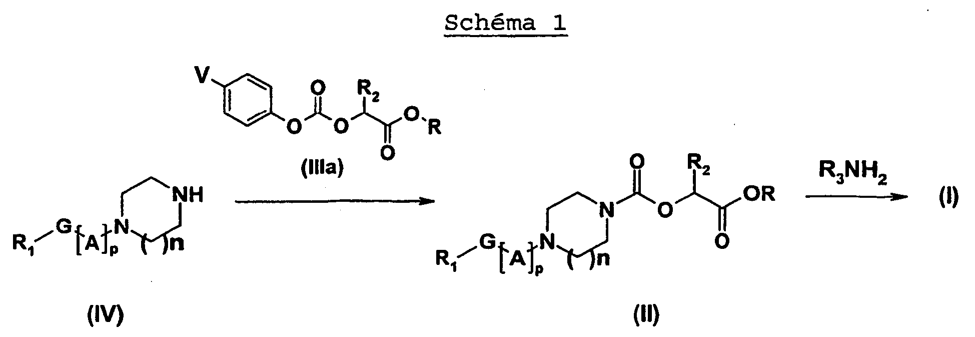

Une autre méthode (schéma 2) d'obtention des composés de formule générale (I) consiste à faire réagir un dérivé de pipérazine ou d'homopipérazine de formule générale (VII), dans laquelle PG représente un groupe protecteur tel qu'un *tert*-butyloxycarbonyl (Boc), avec un carbonate de formule générale (IIIb) dans laquelle V représente un atome d'hydrogène ou un groupe nitro et R₂ et R₃ sont tels que définis dans la formule générale (I), puis à déprotéger le composé résultant, par exemple en présence d'une solution d'acide chlorhydrique dans un solvant tel que l'isopropanol. Le carbamate-amide de formule générale (V) ainsi obtenu est ensuite transformé en composé de formule générale (I), par réaction avec un dérivé de formule générale (VI) dans laquelle R₁, G, p et A sont tels que définis dans la formule générale (I) et W représente un atome de chlore, brome ou iode, ou un groupe mésylate ou tosylate. La réaction de N-alkylation peut être réalisée dans un solvant tel que l'acétonitrile ou le toluène, en présence d'une base telle que le carbonate de potassium ou la diisopropyléthylamine.

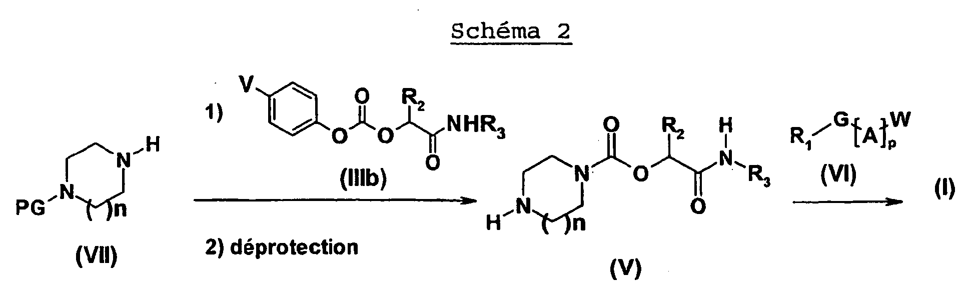

Les composés de formule générale (I), (II) et (IV), dans laquelle R₁ représente un groupe de type aryle-aryle, aryle-hétéroaryle, hétéroaryle-aryle ou hétéroaryle-hétéroaryle, peuvent être également préparés par réaction des composés de formule générale (I), (II) ou (IV) correspondants, pour lesquels R₄ est substitué par un atome de chlore, de brome, d'iode ou par un groupement triflate dans la position où le groupe R₆ doit être introduit, avec un dérivé d'acide aryl- ou hétéroaryl-boronique suivant les conditions de réaction de Suzuki (Chem. Rev. 1995, 95, 2457-2483) ou avec un dérivé d'aryl- ou d'hétéroaryl-tri-alkylstannane suivant les conditions de réaction de Stille (Angew. Chem. Int. Ed. 1986, 25, 504-524).

Les carbonates de formule générale (IIIa) et (IIIb) peuvent être préparés selon toute méthode décrite dans la littérature, par exemple par réaction d'un alcool de formule générale respective HOCHR₂COOR où R représente un groupe méthyle ou éthyle, ou HOCHR₂CONHR₃ où R₃ est tel que défini dans la formule générale (I), avec le chloroformiate de phényle ou de 4-nitrophényle, en présence d'une base telle que la triéthylamine ou la diisopropyléthylamine.

Les composés de formule générale (IV), (VI) et (VII) ainsi que les amines de formule générale R₃NH₂, quand leur mode de préparation n'est pas décrit, sont disponibles dans le commerce ou décrits dans la littérature, ou peuvent être préparés selon différentes méthodes décrites dans la littérature ou connues de l'homme de métier.

L'invention, selon un autre de ses aspects, a également pour objet les composés de formule (II) et (V). Ces composés sont utiles comme intermédiaires de synthèse des composés de formule (I).

Les exemples qui suivent illustrent la préparation de quelques composés de l'invention. Ces exemples ne sont pas limitatifs et ne font qu'illustrer l'invention. Les microanalyses, les spectres I.R. et R.M.N. et/ou la LC-MS (Liquid Chromatography coupled to Mass Spectroscopy) confirment les structures et les puretés des composés obtenus.
PF(°C) représente le point de fusion en degrés Celsius.
Les numéros indiqués entre parenthèses dans les titres des exemples correspondent à ceux de la 1ère colonne du tableau ci-après.

### Exemple 1 (composé n° 85)

### trans-4-(3-phénylprop-2-en-1-yl)pipérazine-1-carboxylate de 2-(méthylamino)-2-oxoéthyle

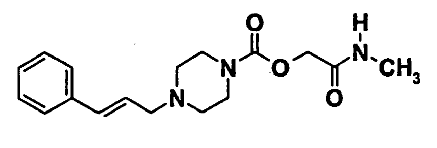

### 1.1. trans - 4-(3-phénylprop-2-en-1-yl)pipérazine-1-carboxylate de 2-(éthoxy)-2-oxoéthyle

On chauffe à 80°C pendant une nuit une solution de 1,40 g (6,93 mmoles) de *trans*-1-cinnamylpipérazine et de 1,74 g (7,76 mmoles) de {[(phénoxy)carbonyl]oxy}acétate d'éthyle (J. Med. Chem., 1999, 42, 277-290) dans 15 ml de toluène. On évapore à sec et on reprend avec 50 ml d'acétate d'éthyle. On lave avec 2 fois 20 ml d'eau et 1 fois 10 ml d'une solution aqueuse saturée en chlorure de sodium. On sèche sur sulfate de sodium et on évapore à sec. On purifie le résidu par chromatographie sur gel de silice en éluant par un mélange 50/50 de cyclohexane et d'acétate d'éthyle, puis par de l'acétate d'éthyle pour obtenir 0,814 g de produit sous forme d'une huile jaune-pâle.

### 1.2. trans-4-(3-phénylprop-2-en-1-yl)pipérazine-1-carboxylate de 2-(méthylamino)-2-oxoéthyle

On dissout 0,8 g (2,4 mmoles) de trans-4-(3-phénylprop-2-en-1-yl)pipérazine-1-carboxylate de 2-(éthoxy)-2-oxoéthyle, obtenu à l'étape 1.1., dans 10 ml d'une solution 2M de méthylamine (20 mmoles) dans le méthanol. On laisse réagir une heure et demie à température ambiante puis on évapore à sec. On purifie le résidu par chromatographie sur gel de silice en éluant d'abord par de l'acétate d'éthyle, puis par un mélange 90/10 d'acétate d'éthyle et de méthanol. On obtient 0,548 g de poudre blanche
Point de fusion (°C) : 109 - 111
LC-MS : M+H = 318
RMN-¹H (DMSO-d₆) : δ (ppm) : 7,80 (s large, 1H); 7,50 - 7,15 (m, 5H) ; 6,55 (d, 1H) ; 6,25 (td, 1H); 4,40 (s, 2H); 3,40 (m, 4H); 3,10 (d, 2H); 2,60 (d, 3H); 2,40 (m, 4H).

### Exemple 2 (composé n° 99)

### 4-{3-[3-(trifluorométhyl)phényl]prop-2-yn-1-yl}-1,4-diazepane-1-carboxylate de 2-amino-2-oxoéthyle

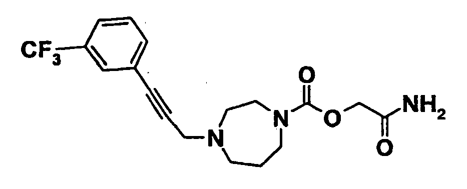

### 2.1. 4-{3-[3-(trifluorométhyl)phényl]prop-2-yn-1-yl}-1,4-diazepane-1-carbaldéhyde

On chauffe à 80°C un mélange de 1,28 g (10 mmoles) de 1,4-diazepane-1-carbaldéhyde et de 0,33 g (11 mmoles) de paraformaldéhyde dans 13 ml de dioxane jusqu'à obtention d'une solution homogène. On ajoute 1,70 g (10 mmoles) de 3-trifluorométhylphénylacétylène en solution dans 7 ml de dioxane et 1,81 g (10 mmoles) de diacétate de cuivre. On chauffe à 80°C pendant 4 heures. On refroidit à température ambiante et on dilue par 75 ml d'acétate d'éthyle. On lave la phase organique par 25 ml d'une solution d'ammoniaque à 30% puis par une solution aqueuse saturée en chlorure de sodium. On sèche sur sulfate de sodium et on évapore à sec. On purifie le résidu par chromatographie sur gel de silice en éluant par un mélange 98/2/0,2 puis 96/4/0,4 et 94/6/0,6 de dichlorométhane, de méthanol et d'ammoniaque à 30% pour obtenir 2,67 g de produit sous forme d'huile jaune.

### 2.2. 4-{3-[3-(trifluorométhyl)phényl]prop-2-yn-1-yl}-1,4-diazepane

On dissout 2,63 g (8,48 mmoles) de 4-{3-[3-(trifluorométhyl)phényl]prop-2-yn-1-yl}-1,4-diazepane-1-carbaldéhyde obtenu à l'étape 2.1. dans 7,5 ml de méthanol. On ajoute 3,5 ml d'une solution aqueuse d'hydroxyde de sodium (30 mmoles) à 35% et on chauffe à reflux pendant 3 heures. On refroidit à température ambiante. On dilue par 20 ml d'eau et 75 ml de dichlorométhane. On décante puis on extrait la phase aqueuse par 2 fois 25 ml de dichlorométhane. On lave les phases organiques par 25 ml d'eau puis par 25 ml d'une solution aqueuse saturée en chlorure de sodium. On sèche sur sulfate de sodium et on évapore à sec pour obtenir 2,25 g de produit sous forme d'huile rouge, utilisée tel quelle dans l'étape suivante.

### 2.3. 4-{3-[3-(trifluorométhyl)phényl]prop-2-yn-1-yl}-1,4-diazepane-1-carboxylate de 2-(éthyloxy)-2-oxoéthyle

On chauffe à 60°C pendant une nuit une solution de 2,25 g (7,95 mmoles) de 4-{3-[3-(trifluorométhyl)phényl]prop-2-yn-1-yl}-1,4-diazepane obtenu à l'étape 2.2. et de 2,68 g (11,9 mmoles) de {[(phényloxy)carbonyl]oxy}-acétate d'éthyle dans 10 ml de toluène. On ajoute 5 g de silice et on évapore à sec. On purifie le résidu par chromatographie sur gel de silice en éluant par un mélange 60/40 puis 40/60 de cyclohexane et d'acétate d'éthyle puis par de l'acétate d'éthyle pour obtenir 2,42 g de produit sous forme d'huile orange.

### 2.4. 4-{3-[3-(trifluorométhyl)phényl]prop-2-yn-1-yl}-1,4-diazepane-1-carboxylate de 2-amino-2-oxoéthyle

On dissout 0,77 g (1,87 mmole) de 4-{3-[3-(trifluorométhyl)phényl]prop-2-yn-1-yl}-1,4-diazepane-1-carboxylate de 2-(éthyloxy)-2-oxoéthyle obtenu à l'étape 2.3., dans 14 ml d'une solution 7 M d'ammoniac (98 mmoles) dans le méthanol. On laisse réagir une nuit à température ambiante puis on ajoute 2 g de silice et on évapore à sec. On purifie le résidu par chromatographie sur gel de silice en éluant par un mélange 97/3/0,3 puis 95/5/0,5 et 93/7/0,7 de dichlorométhane, de méthanol et d'ammoniaque à 30%. On recristallise ensuite dans un mélange d'acétate d'éthyle et de diisopropyléther pour obtenir 0,57 g de cristaux blancs.
Point de fusion (°C) : 102-104
LC-MS : M+H = 384
RMN-¹H (CDCl₃) δ (ppm) : 7, 70 (s, 1H); 7, 55 (m, 2H); 7, 45 (d, 1H); 6,15 (m large, 1H); 5,50 (m large, 1H); 4,65 (s, 2H); 3,65 (m+s, 6H); 2, 85 (m, 4H); 1, 95 (m, 2H).

### Exemple 3 (composé n° 130)

### 4-{2-[(4-chlorophényl)oxy]éthyl}pipérazine-1-carboxylate de 2-(méthylamino)-2-oxoéthyle

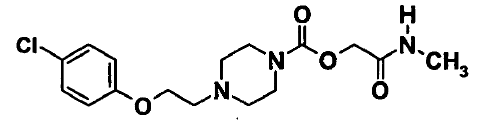

### 3.1. carbonate de 4-nitrophényle et de 2-(méthylamino)-2-oxoéthyle

A une suspension de 2,62 g (29,4 mmoles) de 2-hydroxy-N-méthylacétamide et de 16,5 g (58,7 mmoles) de diisopropyléthylamine supportée (Ps-DIEA d'Argonaut, charge = 3,56 mmoles/g) dans 250 ml de dichlorométhane, on ajoute par petites portions et à température ambiante 5,93 g (29,4 mmoles) de chloroformiate de 4-nitrophényle. On poursuit l'agitation orbitalaire à température ambiante pendant 16 heures. On filtre la résine, on rince avec 150 ml de dichlorométhane et on concentre le filtrat sous pression réduite. On obtient 6 g de produit sous forme de solide jaune clair que l'on utilise tel quel dans l'étape suivante.

### 3.2. pipérazine-1,4-dicarboxylate de 1,1-diméthyléthyle et de 2-(méthylamino)-2-oxoéthyle

A une solution refroidie à 0°C de 1,1 g (3 mmoles) de carbonate de 4-nitrophényle et de 2-(méthylamino)-2-oxoéthyle préparé à l'étape 3.1., dans 10 ml de 1,2-dichloroéthane, on ajoute goutte à goutte, à environ 0°C, une solution de 0,53 g (2,85 mmoles) de pipérazine-1-carboxylate de 1,1-diméthyléthyle dans 5 ml de 1,2-dichloroéthane. On poursuit l'agitation à 0°C pendant 1 heure, puis à température ambiante pendant 3 heures.
On concentre sous pression réduite. On purifie le résidu par chromatographie sur gel de silice en éluant avec un mélange 20/80 d'acétate d'éthyle et de cyclohexane puis on augmente progressivement le gradient pour terminer l'élution avec l'acétate d'éthyle. On triture dans le diisopropyléther pour obtenir 0,61 g de produit sous forme de solide blanc utilisé tel quel dans l'étape suivante.

### 3.3. chlorhydrate de pipérazine-1-carboxylate de 2-(méthylamino)-2-oxoéthyle

A une solution de 2,68 g (8,9 mmoles) de pipérazine-1,4-dicarboxylate de 1,1-diméthyléthyle et de 2-(méthylamino)-2-oxoéthyle, obtenu selon l'étape 3.2., dans 25 ml de dichlorométhane, on ajoute 25 ml d'une solution d'acide chlorhydrique 6 N dans l'isopropanol. On poursuit l'agitation à température ambiante pendant 1 heure. On sépare la phase organique par filtration à travers une cartouche hydrophobe et on concentre sous pression réduite. Après trituration dans l'isopropanol, on obtient 2,05 g de produit.
Point de fusion (°C) : 167-169°C

### 3.4. 4-{2-[(4-chlorophényl)oxy]éthyl}pipérazine-1-carboxylate de 2-(méthylamino)-2-oxoéthyle

On chauffe à 85°C pendant 16 heures, une solution de 0,073 g (0,3 mmole) de chlorhydrate de pipérazine-1-carboxylate de 2-(méthylamino)-2-oxoéthyle préparé à l'étape 3.3., 0,13 g (0,9 mmole) de carbonate de potassium et 0,069 g (0,29 mmole) de 1-(2-bromo-éthoxy)-4-chlorobenzène dans 3 ml d'acétonitrile. Après refroidissement à température ambiante, on filtre les minéraux à travers une cartouche munie d'un fritté et contenant de la célite. On rince avec de l'acétone et on concentre sous pression réduite. Après chromatographie sur gel de silice en éluant avec un mélange de 95/5 de dichlorométhane et de méthanol, suivie d'une cristallisation dans le diisopropyléther, on obtient 0,089 g de produit sous forme de solide blanc.
LC-MS: M+H = 356
Point de fusion: 159-161°C
RMN ¹H (CDCl₃) δ (ppm) : 7,25 (dd, 2H); 6,85 (dd, 2H); 6,05 (large s, 1H) ; 4, 60 (s, 2H) ; 4,10 (t, 2H) ; 3, 55 (m, 4H) ; 2,90 (d, 3H); 2,85 (t, 2H); 2,60 (m, 4H).

### Exemple 4 (composé n° 25)

### 4-(2-naphtalèn-2-yléthyl)pipérazine-1-carboxylate de 2-(méthylamino)-2-oxoéthyle

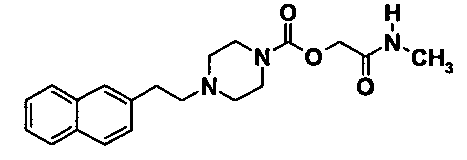

A une solution refroidie à 0°C de 0,13 g (0,75 mmole) de 2-naphtalène-2-yléthanol et de 0,19 ml (1,13 mmole) de diisopropyléthylamine dans 7,5 ml de dichlorométhane, on ajoute 0,07 ml (0,9 mmole) de chlorure de méthanesulfonyle. On poursuit l'agitation à froid pendant 0,5 heure, puis à température ambiante pendant 2 heures. On concentre sous pression réduite.
On reprend le résidu dans 5 ml d'acétonitrile, on ajoute 0,12 g (0,5 mmole) de chlorhydrate de pipérazine-1-carboxylate de 2-(méthylamino)-2-oxoéthyle préparé suivant l'exemple 3.3., et 0,20 g (1,5 mmole) de carbonate de potassium. On chauffe à 70°C pendant 16 heures. Après refroidissement à température ambiante, on concentre sous pression réduite. Le résidu est mis en suspension dans du dichlorométhane et on lave avec une solution saturée de bicarbonate de sodium puis avec de l'eau. On récupère la phase organique par filtration sur une membrane hydrophobe et on concentre sous pression réduite. Après chromatographie sur gel de silice en éluant avec un mélange de 95/5 de dichlorométhane et de méthanol, suivie d'une cristallisation dans le diisopropyléther, on obtient 0,069 g de produit sous forme de solide blanc. LC-MS: M+H = 356
Point de fusion: 133-135°C
RMN ¹H (CDCl₃) δ (ppm) : 7,85 (m, 3H); 7,65 (s, 1H); 7,55-7,30(m, 3H) ; 6,05 (large s, 1H) ; 4,60 (s, 2H) ; 3,55 (m, 4H); 3,05-2,65 (m, 7H); 2,55 (m, 4H).

### Exemple 5 (composé n°50)

### Chlorhydrate de 4-(3-biphényl-3-yl-1,1-dimethylpropyl)pipérazine-1-carboxylate de 2-(méthylamino)-2-oxoéthyle

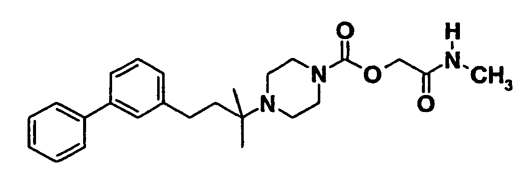

### 5.1. 1-(2,2-diméthylpropanoyl)-4-(1,1-diméthylprop-2-yn-1-yl)pipérazine

On dissout 0,756 g (6 mmoles) d'acétate de 1,1-diméthylprop-2-yn-1-yle (J. Org. Chem.1994, 59, 2282-2284) et 2,235 g (12 mmoles) de pipérazine-1-carboxylate de 1,1-diméthyléthyle dans 9 mL de tétrahydrofurane puis on ajoute 0,059 g (0,6 mmole) de chlorure cuivreux. On chauffe à reflux pendant 3 heures. Après refroidissement à température ambiante, on ajoute 100 mL d'acétate d'éthyle, 10 mL d'hydroxyde de sodium aqueux 1N et 2 mL d'ammoniaque à 30%. On décante la phase organique, on la lave par 2 fois 10 mL d'eau puis par 10 mL d'une solution aqueuse saturée en chlorure de sodium. On sèche sur sulfate de sodium et on évapore. On purifie le produit par chromatographie sur gel de silice en éluant par un mélange 85/15 puis 75/25 et 65/35 de cyclohexane et d'acétate d'éthyle pour obtenir 1,19 g (4,71 mmoles) de produit sous forme de solide jaune pâle.
Point de fusion: 106-109°C

### 5.2. 1-(3-biphényl-3-yl-1,1-diméthylprop-2-yn-1-yl)-4-(2,2-diméthylpropanoyl)pipérazine

On dissout 1,05 g (4,5 mmoles) de 3-bromo-biphényle et 0,9 g (3,6 mmoles) de 1-(2,2-diméthylpropanoyl)-4-(1,1-diméthylprop-2-yn-1-yl)pipérazine, préparé à l'étape 5.1., 0,75 mL (5,38 mmoles) de triéthylamine et 0,028 g (0,11 mmole) de triphénylphosphine dans 8 mL de tétrahydrofurane. Sous atmosphère d'argon, on ajoute 0,126 g (0,18 mmole) du complexe dichlorure de bis(triphénylphosphine) de palladium. On agite 15 minutes puis on ajoute 0,014 g (0,07 mmole) d'iodure cuivreux. On agite à température ambiante pendant 4 heures puis à 60°C pendant la nuit. Après refroidissement à température, on dilue par 25 mL d'acétate d'éthyle et on filtre sur papier. On rince le solide par 4 fois 10 mL d'acétate d'éthyle. On ajoute 4 g de silice au filtrat et on évapore à sec. On purifie par chromatographie sur gel de silice en éluant par un mélange 90/10 puis 80/20 et 70/30 de cyclohexane et d'acétate d'éthyle pour obtenir 0,90 g (2,22 mmoles) de produit sous forme d'huile orange.

### 5.3. 4-(3-biphényl-3-yl-1,1-diméthylpropyl)pipérazine-1-carboxylate de 1,1-diméthyléthyle

On dissout 0,87 g (2,15 mmoles) de 1-(3-biphényl-3-yl-1,1-diméthylprop-2-yn-1-yl)-4-(2,2-diméthylpropanoyl)pipérazine, préparé à l'étape 5.2., dans un mélange de 5 mL de méthanol et 15 mL d'acétate d'éthyle. On ajoute 0,2 g d'oxyde de platine et on agite sous une atmosphère d'hydrogène à 40 Psi pendant 6 heures. On filtre sur papier et on rince par 3 fois 10 mL d'acétate d'éthyle. On ajoute 2 g de silice au filtrat et on évapore à sec. On purifie par chromatographie sur gel de silice en éluant par un mélange 90/10 puis 85/15 et 80/20 de cyclohexane et d'acétate d'éthyle pour obtenir 0,36 g (0,88 mmole) de produit sous forme d'huile incolore.

### 5.4. 1-(3-biphényl-3-yl-1,1-diméthylpropyl)pipérazine

On ajoute 0,65 mL (8,4 mmoles) d'acide trifluoroacétique à une solution de 0,35 g (0,86 mmole) de 4-(3-biphényl-3-yl-1,1-diméthylpropyl)pipérazine-1-carboxylate de 1,1-diméthyléthyle, préparé à l'étape 5.3., dans 5 mL de dichlorométhane. On agite 2 heures puis on rajoute 0,65 mL d'acide trifluoroacétique. On agite 2 heures supplémentaires puis on dilue par 10 mL de 1,2-dichloroéthane et on évapore à sec. On reprend le résidu par un mélange de 50 mL de dichlorométhane et de 20 mL d'une solution aqueuse à 15% d'hydroxyde de sodium. On décante et on extrait la phase aqueuse par 2 fois 20 mL de dichlorométhane.

Les phases organiques sont lavées par 10 mL d'eau puis par 20 mL d'une solution aqueuse saturée en chlorure de sodium, séchées sur sulfate de sodium et évaporées pour fournir 0,25 g (0,81 mmole) de produit sous forme d'huile jaune.

### 5.5. Chlorhydrate de 4-(3-biphényl-3-yl-1,1-dimethylpropyl)pipérazine-1-carboxylate de 2-(méthylamino)-2-oxoéthyle

On chauffe à 60°C pendant une nuit une solution de 0,25 g (0,81 mmole) de 1-(3-biphényl-3-yl-1,1-diméthylpropyl)pipérazine, préparé à l'étape 5.4., et de 1,5 g (1,22 mmole) de {[(phényloxy)carbonyl]oxy}acétate d'éthyle puis on évapore à sec. On redissout le résidu dans un mélange de 4 mL d'une solution de méthylamine 2M (8 mmoles) dans le tétrahydrofurane et de 2 mL de méthanol. On laisse réagir une nuit puis on ajoute 1 g de silice et on évapore. On purifie le produit par chromatographie sur gel de silice en éluant par un mélange 98/2 puis 96/4 et 94/6 de dichlorométhane et de méthanol pour obtenir 0,23 g (0,54 mmole) de produit sous forme de gomme incolore.
On redissout le produit dans 5 mL d'acétate d'éthyle et on ajoute 1 mL d'une solution d'acide chlorhydrique 5N dans l'isopropanol. On évapore à sec. On reprend le résidu dans 15 mL d'acétate d'éthyle chaud. On filtre le solide, on le rince par 2 fois 3 mL d'acétate d'éthyle et on le sèche pour obtenir 0,215 g (0,46 mmole) de produit sous forme de poudre blanche.
LC-MS: M+H = 424
Point de fusion: 212-216°C (déc.)
RMN ¹H (CDCl₃) δ (ppm) : 12,50 (large s, 1H) ; 7,55 (d, 2H); 7,40 (m, 6H); 7,20 (d, 1H); 6,05 (large s, 1H); 4,60 (s, 2H); 4,30-4,10 (m, 4H); 3,55 (large d, 2H) ; 3,05-2,75 (m+d, 5H) ; 2,15 (m, 2H); 1, 70 (s, 8H).

### Exemple 6 (composé n° 29)

### 4-{2-[3-(4-chlorophényl)isoxazol-5-yl]éthyl}pipérazine-1-carboxylate de 2-(méthylamino)-2-oxoéthyle

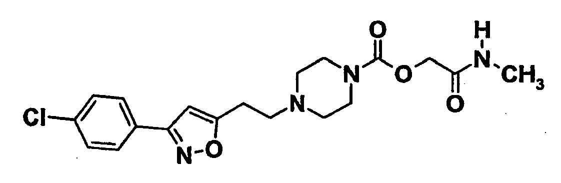

### 6.1. 2-[3-(4-chlorophényl)isoxazol-5-yl]éthanol

On ajoute goutte à goutte 1,63 mL (11,58 mmoles) de triéthylamine à une solution de 1,18 mL (15,57 mmoles) de but-4-yn-1-ol et de 2,0 g (10,52 mmoles) de chlorure de 4-chloro-N-hydroxybenzènecarboximidoyle (J. Med. Chem. 1998, 41, 4556-4566) dans 30 mL de dichlorométhane refroidie par un bain de glace. On laisse réagir une nuit à température ambiante. On ajoute 50 mL de dichlorométhane et on lave par 2 fois 50 mL d'eau puis par 50 mL d'une solution aqueuse saturée en chlorure de sodium. On sèche sur sulfate de sodium et on évapore. On purifie le résidu par chromatographie sur gel de silice en éluant par un mélange 80/20 puis 70/30 de cyclohexane et d'acétate d'éthyle pour obtenir 1,1 g (4,91 mmoles) de produit sous forme de solide blanc.
Point de fusion: 65-67°C

### 6.2. 4-{2-[3-(4-chlorophényl)isoxazol-5-yl]éthyl}pipérazine-1-carboxylate de 2-(méthylamino)-2-oxoéthyle

A une solution de 0,100 g (0,447 mmole) de 2-[3-(4-chlorophényl)isoxazol-5-yl]éthanol, préparé à l'étape 6.1., et de 0,082 ml (0,47 mmole) de diisopropyléthylamine dans 5 ml de dichlorométhane, on ajoute 0,036 ml (0,469 mmole) de chlorure de méthanesulfonyle. On agite à température ambiante pendant 4 heures, puis on lave avec une solution aqueuse saturée de chlorure d'ammonium et une solution aqueuse saturée de chlorure de sodium. On concentre sous pression réduite. On reprend le résidu dans 5 ml d'acétonitrile, on ajoute 0,107 g (0,45 mmole) de chlorhydrate de pipérazine-1-carboxylate de 2-(méthylamino)-2-oxoéthyle préparé suivant l'exemple 3.3, et 0,186 g (1,35 mmole) de carbonate de potassium. On chauffe à 75°C pendant 16 heures. Après refroidissement à température ambiante, on concentre sous pression réduite. On reprend le résidu dans de l'acétate d'éthyle et on lave avec de l'eau puis avec une solution aqueuse saturée en chlorure de sodium. On évapore et on purifie le résidu par chromatographie sur gel de silice en éluant par du dichlorométhane puis par un mélange 90/10 de dichlorométhane et de méthanol. On obtient 0,054 g (0,132 mmole) de produit sous forme de solide blanc.
LC-MS : M+H = 407
Point de fusion: 130-132°C
RMN ¹H (DMSO-d₆) δ (ppm) : 7,85 (d, 2H); 7,75 (massif, 1H); 7,55 (d, 2H); 6,85 (s, 1H); 4,40 (s, 2H); 3,40 (m, 4H); 2,95 (t, 2H) ; 2,70 (t, 2H) ; 2,55 (d, 3H) ; 2,40 (m, 4H).

### Exemple 7 (composé n° 52)

### 4-[3-(3'-Chloro-biphényl-3-yl)propyl]pipérazine-1-carboxylate de 2-(méthylamino)-2-oxéthyle

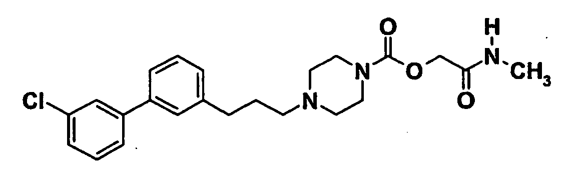

### 7.1. 3-(3-Bromo-phényl)propan-1-ol

A une suspension de 1,84 g (8 mmoles) d'acide 3-(3-bromo-phényl)propionique et de 0,91 g (24 mmoles) borohydrure de sodium dans 20 ml de THF, refroidie à 0°C, on ajoute par petites portions 3,2 ml (25 mmoles) du complexe trifluoroborane-diéthyléther. On poursuit l'agitation à froid pendant 1 heure, puis à température ambiante pendant 16 heures. On refroidit le milieu réactionnel à 0°C et on le neutralise à pH 7~8 par addition d'une solution 1N d'hydroxyde de sodium aqueux. On concentre sous pression réduite puis on reprend le résidu dans de l'eau. On extrait avec du dichlorométhane et on sèche sur sulfate de sodium. Après filtration, la phase organique est concentrée sous pression réduite. On obtient 1,62 g (7,53 mmoles) de produit sous forme d'huile que l'on utilise tel quel dans l'étape suivante.

### 7.2. 4-[3-(3-Bromo-phényl)propyl]pipérazine-1-carboxylate de 2-(méthylamino)-2-oxéthyle

A une solution de 1,57 g (6,7 mmoles) de 3-(3-bromo-phényl)propan-1-ol préparé à l'étape 7.1 et de 1,73 ml (10,1 mmoles) de diisopropyléthylamine dans 38 ml de dichlorométhane, refroidie à 0°C on ajoute 0,63 ml (8,14 mmoles) de chlorure de méthanesulfonyle. On poursuit l'agitation à froid pendant 0,5 heure puis à température ambiante pendant 2 heures. On concentre sous pression réduite puis on met le résidu en suspension dans 35 ml d'acétonitrile. On ajoute 1,34 g (5,35 mmoles) de chlorhydrate de pipérazine-1-carboxylate de 2-(méthylamino)-2-oxoéthyle préparé suivant l'exemple 3.3., et 2,2 g (16 mmoles) de carbonate de potassium. On chauffe à 75°C pendant 16 heures. Après refroidissement à température ambiante, on concentre sous pression réduite puis on reprend le résidu dans de l'eau. On extrait avec de l'acétate d'éthyle et on sèche sur sulfate de sodium. Après filtration, la phase organique est concentrée sous pression réduite. On purifie par chromatographie sur gel de silice en éluant avec un mélange 98/2 de dichlorométhane et de méthanol. Après cristallisation dans le diisopropyléther, on obtient 0,84 g (2,10 mmoles)de cristaux blancs.

### 7.3. 4-[3-(3'-Chloro-biphényl-3-yl)propyl]pipérazine-1-carboxylate de 2-(méthylamino)-2-oxéthyle

A une suspension de 0,14 g (0,35 mmole) de 4-[3-(3-bromo-phenyl)propyl]pipérazine-1-carboxylate de 2-(méthylamino)-2-oxéthyle préparé à l'étape 7.2., dans un mélange de 4 ml de toluène et 0,6 ml d'éthanol, on ajoute 0,08 g (0,07 mole) du complexe tetrakis(triphénylphospine) de palladium, 1,05 ml (2,1 mmoles) d'une solution aqueuse 2M de carbonate de sodium et 0,22 g (1,4 mmole) d'acide 3-chloro-benzeneboronique. On chauffe à 150°C sous irradiations micro-ondes pendant 5 minutes et on récupère la phase organique par filtration sur une cartouche munie d'un fritté et contenant de la célite et du sulfate de sodium. On rince avec du toluène et on concentre le filtrat sous pression réduite. On purifie le produit par chromatographie sur gel de silice en éluant avec un mélange 90/10 d'acétate d'éthyle et de méthanol. On reprend ensuite dans du n-heptane pour obtenir 0,086 g (0,18 mmole) de produit sous forme de cristaux blancs.
LC-MS: M+H = 430
Point de fusion: 82-85°C
RMN ¹H δ (ppm): 7,35 (m, 8H) ; 6,05 (large s, 1H) ; 4,6 (s, 2H); 3,55 (m, 4H); 2,85 (d, 3H); 2,75 (t, 2H); 2,45 (m, 6H); 1,9 (m, 2H).

Le tableau 1 qui suit illustre les structures chimiques et les propriétés physiques de quelques composés selon l'invention. Dans la colonne « base ou sel », « base » représente un composé sous forme de bas libre, alors que « HCl » représente un composé sous forme de chlorhydrate.

**Tableau 1**

| | | | | | | | | |
|---|---|---|---|---|---|---|---|---|
| 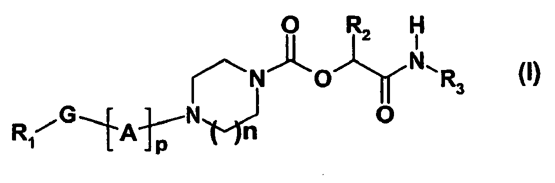 | | | | | | | | |

| **N°** | **R₁** | **G** | **[A]ₚ** | **n** | **R₂** | **R₃** | **PF (°C) (ou M+H)** | **base ou sel** |
|---|---|---|---|---|---|---|---|---|
| 1. | 2-F-phényl | liaison | CH₂ | 1 | H | CH₃ | 196-200 | HCl |
| 2. | 2-Cl-phényl | liaison | CH₂ | 1 | H | CH₃ | 212-217 | HCl |
| 3. | 3-F-phény | liaison | CH₂ | 1 | H | CH₃ | 161-166 | HCl |
| 4. | 3-I-phényl | liaison | CH₂ | 1 | H | CH₃ | (418) | base |
| 5. | 3-Cl-phényl | liaison | CH₂ | 1 | H | CH₃ | 203-207 | HCl |
| 6. | 4-Cl-phényl | liaison | CH₂ | 1 | H | CH₃ | 112-115 | HCl |
| 7. | 4-CH₃O-phényl | liaison | CH₂ | 1 | H | CH₃ | 155-159 | HCl |
| 8. | 4-(phénylCH₂O)-phényl | liaison | CH₂ | 1 | H | CH₃ | 172-178 | HCl |
| 9. | 4-(CH₃)₂CH-phényl | liaison | CH₂ | 1 | H | CH₃ | 104-108 | HCl |
| 10. | 3-phényl-phényl | liaison | CH₂ | 1 | H | CH₃ | 111-114 | HCl |
| 11. | 4-phényl-phényl | liaison | CH₂ | 1 | H | CH₃ | 173-179 | HCl |
| 12. | naphtalèn-1-yl | liaison | CH₂ | 1 | H | CH₃ | 143-145 | base |
| 13. | naphtalèn-2-yl | liaison | CH₂ | 1 | H | CH₃ | 184-186 | HCl |
| 14. | phényl | liaison | (CH₂)₂ | 1 | H | CH₃ | 167-169 | base |
| 15. | 3-Br-phényl | liaison | (CH₂)₂ | 1 | H | CH₃ | (384) | base |
| 16. | 4-Br-phényl | liaison | (CH₂)₂ | 1 | H | CH₃ | (384) | base |
| 17. | 4-CH₃O-phényl | liaison | (CH₂)₂ | 1 | H | CH₃ | 124-126 | base |
| 18. | 3-phényl-phényl | liaison | (CH₂)₂ | 1 | H | CH₃ | 118-120 | base |
| 19. | 4-phényl-phényl | liaison | (CH₂)₂ | 1 | H | CH₃ | 148-150 | base |
| 20. | naphtalèn-1-yl | liaison | (CH₂)₂ | 1 | H | H | 125-127 | base |
| 21. | naphtalèn-1-yl | liaison | (CH₂)₂ | 1 | H | CH₃ | 109-112 | base |
| 22. | naphtalèn-1-yl | liaison | (CH₂)₂ | 1 | H | CH₂CH₃ | 113-115 | base |
| 23. | naphtalèn-1-yl | liaison | (CH₂)₂ | 1 | H | cyclo propyle | 125-127 | base |
| 24. | naphtalèn-1-yl | liaison | (CH₂)₂ | 1 | H | CH₂-cyclo propyle | 113-115 | base |
| 25. | naphtalèn-2-yl | liaison | (CH₂)₂ | 1 | H | CH₃ | 133-135 | base |
| 26. | naphtalèn-2-yl | liaison | (CH₂)₂ | 2 | H | H | 115-119 | base |
| 27. | indol-3-yl | liaison | (CH₂)₂ | 1 | H | CH₃ | 121-123 | base |
| 28. | 3-(4-Cl-phényl)-1*H*-méthyl-pyrazol-5-yl | liaison | (CH₂)₂ | 1 | H | CH₃ | 141-143 | base |
| 29. | 3-(4-Cl-phényl)isoxazol -5-yl | liaison | (CH₂)₂ | 1 | H | CH₃ | 130-132 | base |
| 30. | 5-(4-Cl-phényl)isoxazol -3-yl | liaison | (CH₂)₂ | 1 | H | CH₃ | 146-148 | base |
| 31. | 6-(4-Cl-phényl)pyrimidi n-4-yl | liaison | (CH₂)₂ | 1 | H | CH₃ | 132-134 | base |
| 32. | 1,1-diphénylméthyl | liaison | (CH₂)₂ | 1 | H | CH₃ | 86-88 | base |
| 33. | phényl | liaison | (CH₂)₃ | 1 | H | CH₃ | 315-317 | HCl |
| 34. | 3-Cl-phényl | liaison | (CH₂)₃ | 1 | H | CH₃ | 85-87 | base |
| 35. | 4-Cl-phényl | liaison | (CH₂)₃ | 1 | H | CH₃ | 115-117 | base |
| 36. | 3-Br-phényl | liaison | (CH₂)₃ | 1 | H | CH₃ | (398) | base |
| 37. | 4-Br-phényl | liaison | (CH₂)₃ | 1 | H | CH₃ | (398) | base |
| 38. | 3-CN-phényl | liaison | (CH₂)₃ | 1 | H | CH₃ | 107-109 | base |
| 39. | 3-CF₃-phényl | liaison | (CH₂)₃ | 1 | H | CH₃ | 98-100 | base |
| 40. | 4-CF₃-phényl | liaison | (CH₂)₃ | 1 | H | CH₃ | 85-87 | base |
| 41. | 2-Cl, 4-Cl-phényl | liaison | (CH₂)₃ | 1 | H | CH₃ | 103-105 | base |
| 42. | 2-Cl, 5-Cl-phényl | liaison | (CH₂)₃ | 1 | H | H | 128-130 | base |
| 43. | 2-Cl, 5-Cl-phényl | liaison | (CH₂)₃ | 1 | H | CH₃ | 121-123 | base |
| 44. | pyrimidin-2-yl | liaison | (CH₂)₃ | 1 | H | CH₃ | 103-105 | base |
| 45. | pyrimidin-5-yl | liaison | (CH₂)₃ | 1 | H | CH₃ | 116-118 | base |
| 46. | thiazol-2-yl | liaison | (CH₂)₃ | 1 | H | CH₃ | 83-85 | base |
| 47. | 2-phényl-phényl | liaison | (CH₂)₃ | 1 | H | CH₃ | (396) | base |
| 48. | 3-phényl-phényl | liaison | (CH₂)₃ | 1 | H | CH₃ | 99-101 | base |
| 49. | 4-phényl-phényl | liaison | (CH₂)₃ | 1 | H | CH₃ | 110-113 | base |
| 50. | 3-phényl-phényl | liaison | (CH₂)₂-C(CH₃)₂ | 1 | H | CH₃ | 212-216 | HCl |
| 51. | 4-phényl-phényl | liaison | (CH₂)₂-C(CH₃)₂ | 1 | H | CH₃ | 101-103 | base |
| 52. | 3-(3-Cl-phényl)-phényl | liaison | (CH₂)₃ | 1 | H | CH₃ | 82-85 | base |
| 53. | 3-(4-Cl-phényl)-phényl | liaison | (CH₂)₃ | 1 | H | CH₃ | 136-138 | base |
| 54. | 3-(3-CH₃O)phényl)-phényl | liaison | (CH₂)₃ | 1 | H | CH₃ | (426) | base |
| 55. | 3-(4-CH₃O)phényl)-phényl | liaison | (CH₂)₃ | 1 | H | CH₃ | 135-137 | base |
| 56. | 3-(3-CN-phényl)-phényl | liaison | (CH₂)₃ | 1 | H | CH₃ | 152-154 | base |
| 57. | 3-(4-CN-phényl)-phenyl | liaison | (CH₂)₃ | 1 | H | CH₃ | 137-139 | base |
| 58. | 4-(3-Cl-phényl)-phényl | liaison | (CH₂)₃ | 1 | H | CH₃ | 101-103 | base |
| 59. | 4-(4-Cl-phényl)-phenyl | liaison | (CH₂)₃ | 1 | H | CH₃ | 125-128 | base |
| 60. | 4-(3-CH₃O)phényl)-phényl | liaison | (CH₂)₃ | 1 | H | CH₃ | 97-100 | base |
| 61. | 4-(4-CH₃O)phényl)-phényl | liaison | (CH₂)₃ | 1 | H | CH₃ | 128-130 | base |
| 62. | 4-(3-CN-phényl)-phenyl | liaison | (CH₂)₃ | 1 | H | CH₃ | 108-110 | base |
| 63. | 4-(4-CN-phényl)-phényl | liaison | (CH₂)₃ | 1 | H | CH₃ | 148-150 | base |
| 64. | naphtalèn-1-yl | liaison | (CH₂)₃ | 1 | H | CH₃ | 104-106 | *HCl* |
| 65. | naphtalèn-2-yl | liaison | (CH₂)₃ | 1 | H | CH₃ | 110-112 | base |
| 66. | 3-(4-Cl-phényl)-1*H*-methyl-pyrazol-5-yl | liaison | (CH₂)₃ | 1 | H | CHCH₃ | 157-159 | base |
| 67. | 5-(4-Cl-phényl)isoxazol -3-yl | liaison | (CH₂)₃ | 1 | H | CH₃ | 125-127 | base |
| 68. | 3-(4-Cl-phényl)isoxazol -5-yl | liaison | (CH₂)₃ | 1 | H | H | 132-134 | base |
| 69. | 3-(4-Cl-phényl)isoxazol -5-yl | liaison | (CH₂)₃ | 1 | H | CH₃ | 108-110 | base |
| 70. | 3-(naphtalen-2-yl)isoxazol-5-yl | liaison | (CH₂)₃ | 1 | H | CH₃ | 71-73 | base |
| 71. | 1,1-di-(4-F-phényl)méthyl | liaison | (CH₂)₃ | 1 | H | CH₃ | (446) | base |
| 72. | 3-Cl-phényl | liaison | (CH₂)₄ | 1 | H | CH₃ | 103-105 | base |
| 73. | 4-Cl-phényl | liaison | (CH₂)₄ | 1 | H | CH₃ | 120-122 | base |
| 74. | 3-CN-phényl | liaison | (CH₂)₄ | 1 | H | CH₃ | 127-129 | base |
| 75. | 3-CF₃-phényl | liaison | (CH₂)₄ | 1 | H | CH₃ | 98-100 | base |
| 76. | 4-CF₃-phényl | liaison | (CH₂)₄ | 1 | H | CH₃ | 129-131 | base |
| 77. | pyrimidin-2-yl | liaison | (CH₂)₄ | 1 | H | CH₃ | 141-143 | base |
| 78. | pyrimidin-5-yl | liaison | (CH₂)₄ | 1 | H | CH₃ | 114-116 | base |
| 79. | thiazol-2-yl | liaison | (CH₂)₄ | 1 | H | CH₃ | 93-95 | base |
| 80. | naphtalèn-1-yl | liaison | (CH₂)₄ | 1 | H | CH₃ | 90-92 | base |
| 81. | naphtalèn-2-yl | liaison | (CH₂)₄ | 1 | H | CH₃ | 109-111 | base |
| 82. | 2-phényl-phényl | liaison | (CH₂)₄ | 1 | H | CH₃ | 92-94 | base |
| 83. | 3-phényl-phényl | liaison | (CH₂)₄ | 1 | H | CH₃ | 97-99 | base |
| 84. | phényl | liaison | CH=CHCH₂ *(E)* | 1 | H | H | 115-117 | base |
| 85. | phényl | liaison | CH=CHCH₂ *(E)* | 1 | H | CH₃ | 109-111 | base |
| 86. | 3-Cl-phényl | liaison | C≡CCH₂ | 1 | H | CH₃ | 114-116 | base |
| 87. | 4-Cl-phényl | liaison | C≡CCH₂ | 1 | H | CH₃ | 127-129 | base |
| 88. | 3-CF₃-phényl | liaison | C≡CCH₂ | 1 | H | CH₃ | 131-133 | base |
| 89. | 4-CF₃-phényl | liaison | C≡CCH₂ | 1 | H | CH₃ | 125-127 | base |
| 90. | 3-CN-phényl | liaison | C≡CCH₂ | 1 | H | CH₃ | 134-140 | base |
| 91. | pyrimidin-2-yl | liaison | C≡CCH₂ | 1 | H | CH₃ | 137-139 | base |
| 92. | pyrimidin-5-yl | liaison | C≡CCH₂ | 1 | H | CH₃ | 151-153 | base |
| 93. | thiazol-2-yl | liaison | C≡CCH₂ | 1 | H | CH₃ | 111-113 | base |
| 94. | naphtalèn-1-yl | liaison | C≡CCH₂ | 1 | H | CH₃ | 131-134 | base |
| 95. | naphtalèn-2-yl | liaison | C≡CCH₂ | 1 | H | CH₃ | (366) | base |
| 96. | 2-phényl-phényl | liaison | C≡CCH₂ | 1 | H | CH₃ | (392) | base |
| 97. | 3-phényl-phényl | liaison | C≡CCH₂ | 1 | H | CH₃ | 125-127 | base |
| 98. | 4-phényl-phényl | liaison | C≡CC(CH₃)₂ | 1 | H | CH₃ | 137-139 | base |
| 99. | 3-CF₃-phényl | liaison | C≡CCH₂ | 2 | H | H | 102-104 | base |
| 100. | 3-CF₃-phényl | liaison | C≡CCH₂ | 2 | H | CH₃ | 92-94 | base |
| 101. | 3-Cl-phényl | liaison | C≡C(CH₂)₂ | 1 | H | CH₃ | 115-117 | base |
| 102. | 4-Cl-phényl | liaison | C≡C(CH₂)₂ | 1 | H | CH₃ | 141-143 | base |
| 103. | 3-CF₃-phényl | liaison | C≡C(CH₂)₂ | 1 | H | CH₃ | 93-95 | base |
| 104. | 4-CF₃-phényl | liaison | C≡C(CH₂)₂ | 1 | H | CH₃ | 142-144 | base |
| 105. | 3-CN-phényl | liaison | C≡C(CH₂)₂ | 1 | H | CH₃ | 144-146 | base |
| 106. | pyrimidin-2-yl | liaison | C≡C(CH₂)₂ | 1 | H | CH₃ | 120-122 | base |
| 107. | pyrimidin-5-yl | liaison | C≡C(CH₂)₂ | 1 | H | CH₃ | 159-161 | base |
| 108. | thiazol-2-yl | liaison | C≡C(CH₂)₂ | 1 | H | CH₃ | 103-105 | base |
| 109. | naphtalèn-1-yl | liaison | C≡C(CH₂)₂ | 1 | H | CH₃ | 99-101 | base |
| 110. | naphtalèn-2-yl | liaison | C≡C(CH2)₂ | 1 | H | CH₃ | 140-142 | base |
| 111. | 2-phényl-phényl | liaison | C≡C(CH₂)₂ | 1 | H | CH₃ | (406) | base |
| 112. | 3-phényl-phényl | liaison | C≡C(CH₂)₂ | 1 | H | CH₃ | 102-104 | base |
| 113. | 3-Cl-phényl | liaison | C≡C(CH₂)₃ | 1 | H | CH₃ | 79-81 | base |
| 114. | 4-Cl-phényl | liaison | C≡C(CH₂)₃ | 1 | H | CH₃ | 126-128 | base |
| 115. | 2-F, 4-Cl-phényl | liaison | C≡C(CH₂)₃ | 1 | H | CH₃ | 131-133 | base |
| 116. | 2-Cl, 4-F-phényl | liaison | C≡C(CH₂)₃ | 1 | H | CH₃ | 133-135 | base |
| 117. | 2-Cl, 4-Cl-phényl | liaison | C≡C(CH₂)₃ | 1 | H | CH₃ | 133-135 | base |
| 118. | 2-Cl, 5-Cl-phényl | liaison | C≡C(CH₂)₃ | 1 | H | CH₃ | 110-112 | base |
| 119. | 3-Cl,4-Cl-phényl | liaison | C≡C(CH₂)₃ | 1 | H | CH₃ | 119-121 | base |
| 120. | 3-Cl, 4-F-phényl | liaison | C≡C(CH₂)₃ | 1 | H | CH₃ | 98-100 | base |
| 121. | phényl | O | (CH₂)₂ | 1 | H | CH₃ | 233-235 | base |
| 122. | 2-Cl-phényl | O | (CH₂)₂ | 1 | H | H | 90-92 | base |
| 123. | 2-Cl-phényl | O | (CH₂)₂ | 1 | H | CH₃ | 184-186 | HCl |
| 124. | 2-CN-phényl | O | (CH₂)₂ | 1 | H | CH₃ | 109-111 | base |
| 125. | 3-Cl-phényl | O | (CH₂)₂ | 1 | H | H | > 300 | HCl |
| 126. | 3-Cl-phényl | O | (CH₂)₂ | 1 | H | CH₃ | 105-107 | base |
| 127. | 3-CN-phényl | O | (CH₂)₂ | 1 | H | CH₃ | 141-143 | base |
| 128. | 4-F-phényl | O | (CH₂)₂ | 1 | H | CH₃ | 134-136 | base |
| 129. | 4-Cl-phényl | O | (CH₂)₂ | 1 | H | H | 115-117 | base |
| 130. | 4-Cl-phényl | O | (CH₂)₂ | 1 | H | CH₃ | 159-161 | base |
| 131. | 4-CN-phényl | O | (CH₂)₂ | 1 | H | H | 145-147 | base |
| 132. | 4-CN-phényl | O | (CH₂)₂ | 1 | H | CH₃ | 138-140 | base |
| 133. | 4-(CH₃)₃C-phényl | O | (CH₂)₂ | 1 | H | CH₃ | 111-113 | HCl |
| 134. | 4-CF₃-phényl | O | (CH₂)₂ | 1 | H | H | 104-106 | base |
| 135. | 4-CF₃O-phényl | O | (CH₂)₂ | 1 | H | H | 96-98 | base |
| 136. | 4-CF₃O-phényl | O | (CH₂)₂ | 1 | H | CH₃ | 93-96 | base |
| 137. | 2-Cl, 3-Cl-phényl | O | (CH₂)₂ | 1 | H | H | 136-138 | base |
| 138. | 2-Cl, 3-Cl-phényl | O | (CH₂)₂ | 1 | H | CH₃ | 132-134 | base |
| 139. | 2-Cl, 4-Cl-phényl | O | (CH₂)₂ | 1 | H | H | 178-180 | base |
| 140. | 2-Cl, 4-Cl-phényl | O | (CH₂)₂ | 1 | H | CH₃ | 102-104 | base |
| 141. | 3-Cl, 4-Cl-phényl | O | (CH₂)₂ | 1 | H | CH₃ | 128-130 | base |
| 142. | 3-Cl, 4-Cl-phényl | O | (CH₂)₂ | 1 | H | H | 126-128 | base |
| 143. | 3-Cl, 5-Cl-phényl | O | (CH₂)₂ | 1 | H | CH₃ | 111-113 | base |
| 144. | 3-CF₃, 5-CF₃-phényl | O | (CH₂)₂ | 1 | H | CH₃ | 137-139 | base |
| 145. | 3,4-(OCH₂O)-phényl | O | (CH₂)₂ | 1 | H | CH₃ | 139-141 | base |
| 146. | 3-phényl-phényl | O | (CH₂)₂ | 1 | H | H | 120-122 | HCl |
| 147. | 3-phényl-phényl | O | (CH₂)₂ | 1 | H | CH₃ | 143-145 | HCl |
| 148. | 4-phényl-phényl | O | (CH₂)₂ | 1 | H | H | 238-240 | base |
| 149. | 4-phényl-phényl | O | (CH₂)₂ | 1 | H | CH₃ | 130-132 | base |
| 150. | naphtalèn-1-yl | O | (CH₂)₂ | 1 | H | H | 116-118 | base |
| 151. | naphtalèn-1-yl | O | (CH₂)₂ | 1 | H | CH₃ | 135-137 | base |
| 152. | naphtalèn-2-yl | O | (CH₂)₂ | 1 | H | H | 88-90 | base |
| 153. | naphtalèn-2-yl | O | (CH₂)₂ | 1 | H | CH₃ | 118-120 | base |
| 154. | quinolin-6-yl | O | (CH₂)₂ | 1 | H | H | 203-205 | base |
| 155. | quinolin-6-yl | O | (CH₂)₂ | 1 | H | CH₃ | 126-128 | base |
| 156. | quinolin-8-yl | O | (CH₂)₂ | 1 | H | CH₃ | 99-101 | base |
| 157. | phényl | O | (CH₂)₃ | 1 | H | CH₃ | 103-105 | base |
| 158. | 2-Cl-phényl | O | (CH₂)₃ | 1 | H | CH₃ | 119-121 | base |
| 159. | 3-Cl-phényl | O | (CH₂)₃ | 1 | H | CH₃ | 95-97 | base |
| 160. | 4-Cl-phényl | O | (CH₂)₃ | 1 | H | CH₃ | 116-118 | base |
| 161. | 2-Cl, 3-Cl-phényl | O | (CH₂)₃ | 1 | H | CH₃ | 110-112 | base |
| 162. | 2-Cl,4-Cl-phényl | O | (CH₂)₃ | 1 | H | CH₃ | 115-117 | base |
| 163. | 2-Cl,5-Cl-phényl | O | (CH₂)₃ | 1 | H | CH₃ | 134-136 | base |
| 164. | 2-Cl,6-Cl-phényl | O | (CH₂)₃ | 1 | H | CH₃ | 100-102 | base |
| 165. | 3-Cl,5-Cl-phényl | O | (CH₂)₃ | 1 | H | CH₃ | 121-123 | base |
| 166. | phényl | C=O | (CH₂)₂ | 1 | H | CH₃ | 141-143 | base |
| 167. | 4-Cl-phényl | C=O | (CH₂)₂ | 1 | H | CH₃ | 172-174 | base |
| 168. | phényl | C=O | (CH₂)₃ | 1 | H | CH₃ | 110-112 | base |

Les composés de l'invention ont fait l'objet d'essais pharmacologiques permettant de déterminer leur effet inhibiteur de l'enzyme FAAH (Fatty Acid Amido Hydrolase).

L'activité inhibitrice a été mise en évidence dans un test radioenzymatique basé sur la mesure du produit d'hydrolyse (éthanolamine [1-³H]) de l'anandamide [éthanolamine 1-³H] par la FAAH (Life Sciences (1995), 56, 1999-2005 et Journal of Pharmacology and Experimental Therapeutics (1997), 283, 729-734). Ainsi, les cerveaux de souris (moins le cervelet) sont prélevés et conservés à -80°C. Les homogénats membranaires sont préparés extemporanément par homogénéisation des tissus au Polytron dans un tampon Tris-HCl 10 mM (pH 8) contenant 150 mM NaCl et 1 mM EDTA. La réaction enzymatique est ensuite conduite dans 70 µl de tampon contenant de l'albumine de sérum bovin sans acides gras (1 mg/ml). Sont ajoutés successivement les composés testés à différentes concentrations, l'anandamide [éthanolamine 1-³H] (activité spécifique de 15-20 Ci/mmol) diluée à 10 µM avec de l'anandamide froide et la préparation membranaire (400 µg tissu congelé par essai). Après 15 minutes à 25°C, la réaction enzymatique est arrêtée par addition de 140 µL de chloroforme/méthanol (2 :1). Le mélange est agité 10 minutes puis centrifugé pendant 15 minutes à 3500g. Un aliquot (30 µL) de la phase aqueuse contenant l'éthanolamine [1-³H] est compté par scintillation liquide.
Dans ces conditions, les composés les plus actifs de l'invention présentent des CI₅₀ (concentration inhibant de 50 % l'activité enzymatique contrôle de la FAAH) comprises entre 0,001 et 1 µM.

Le tableau 2 ci-dessous présente les CI₅₀ de quelques composés selon l'invention.

**Tableau 2**

| Composé N° | CI₅₀ |
|---|---|
| 21 | 0, 072 µM |
| 48 | 0, 050 µM |
| 49 | 0, 032 µM |

Il apparaît donc que les composés selon l'invention ont une activité inhibitrice sur l'enzyme FAAH.

L'activité in vivo des composés de l'invention a été évaluée dans un test d'analgésie.
Ainsi, l'administration intrapéritonéale (i.p.) de PBQ (phénylbenzoquinone, 2 mg/kg dans une solution de chlorure de sodium à 0,9 % contenant 5 % d'éthanol) chez des souris mâles OF1 de 25 à 30 g, provoque des étirements abdominaux, en moyenne 30 torsions ou contractions pendant la période de 5 à 15 minutes après injection. Les composés testés sont administrés par voie orale (p.o.) ou par voie intrapéritonéale (i.p.) en suspension dans du Tween 80 à 0,5 %, 60 minutes ou 120 minutes avant l'administration de PBQ. Dans ces conditions, les composés les plus puissants de l'invention réduisent de 35 à 70 % le nombre d'étirements induits par le PBQ, dans une gamme de doses comprise entre 1 et 30 mg/kg.
Par exemple, les composés n° 49 et n° 69 du tableau réduisent, respectivement, de 43% et de 47% le nombre d'étirements induits par le PBQ, à la dose de 10 mg/kg p.o. à 120 minutes.

L'enzyme FAAH (Chemistry and Physics of Lipids, (2000), 108, 107-121) catalyse l'hydrolyse des dérivés endogènes d'amides et d'esters de différents acides gras tels que la *N*-arachidonoyléthanolamine (anandamide), la *N*-palmitoyléthanolamine, la *N*-oléoyléthanolamine, l'oléamide ou le 2-arachidonoylglycérol. Ces dérivés exercent différentes activités pharmacologiques en interagissant, entre autres, avec les récepteurs cannabinoides et vanilloïdes.
Les composés de l'invention, bloquent cette voie de dégradation et augmentent le taux tissulaire de ces substances endogènes. Ils peuvent être utilisés à ce titre dans la prévention et le traitement des pathologies dans lesquelles les cannabinoïdes endogènes et/ou tous autres substrats métabolisés par l'enzyme FAAH, sont impliqués. On peut par exemple citer les maladies et les affections suivantes :
la douleur notamment les douleurs aiguës ou chroniques de type neurogène : migraine, douleurs neuropathiques incluant les formes associées au virus de l'herpès et au diabète, les douleurs aiguës ou chroniques associées aux maladies inflammatoires : arthrite, arthrite rhumatoïde, ostéoarthrite, spondylite, goutte, vascularite, maladie de Crohn, syndrome du colon irritable, les douleurs aiguës ou chroniques périphériques, les vertiges, les vomissements, les nausées en particulier celles consécutives à une chimiothérapie, les troubles du comportement alimentaire en particulier les anorexies et cachexies de diverses natures , les pathologies neurologiques et psychiatriques : tremblements, dyskinésies, dystonies, spasticité, comportements compulsifs et obsessionnels, syndrome de Tourette, toutes les formes de dépression et d'anxiété de toute nature et origine, troubles de l'humeur, psychoses, les maladies neuro-dégénératives aiguës et chroniques : maladie de Parkinson, maladie d'Alzheimer, démence sénile, chorée de Huntington, lésions liées à l'ischémie cérébrale et aux traumatismes crâniens et médullaires, l'épilepsie, les troubles du sommeil incluant les apnées du sommeil, les maladies cardiovasculaires en particulier hypertension, arythmies cardiaques, artériosclérose, crise cardiaque, ischémies cardiaques, l'ischémie rénale, les cancers : tumeurs bénignes de la peau, papillomes et tumeurs cérébrales, tumeurs de la prostate, tumeurs cérébrales (glioblastomes, médullo-épithéliomes, médulloblastomes, neuroblastomes, tumeurs d'origine embryonnaire, astrocytomes, astroblastomes, épendyomes, oligodendrogliomes, tumeur du plexus, neuroépithéliomes, tumeur de l'épiphyse, épendymoblastomes, méningiomes malins, sarcomatoses, mélanomes malins, schwénnomes), les désordres du système immunitaire, notamment les maladies auto-immunes: psoriasis, lupus érythémateux, maladies du tissu conjonctif ou connectivites, syndrome de Sjögren's, spondylarthrite ankylosante, spondylarthrite indifférenciée, maladie de Behcet's, anémies auto-immunes hémolytiques, sclérose en plaques, sclérose latérale amyotrophique, amyloses, rejet de greffes, maladies affectant la lignée plasmocytaire, les maladies allergiques : l'hypersensibilité immédiate ou retardée, rhinites ou conjonctivites allergiques, dermatites de contact, les maladies infectieuses parasitaires, virales ou bactériennes : SIDA, méningites, les maladies inflammatoires, notamment les maladies articulaires : arthrite, arthrite rhumatoïde, ostéoarthrite, spondylite, goutte, vascularite, maladie de Crohn, syndrome du colon irritable, l'ostéoporose, les affections oculaires : hypertension oculaire, glaucome, les affections pulmonaires : maladies des voies respiratoires, bronchospasmes, toux, asthme, bronchite chronique, obstruction chronique des voies respiratoires, emphysème, les maladies gastro-intestinales: syndrome du colon irritable, désordres inflammatoires intestinaux, ulcères, diarrhées, l'incontinence urinaire et l'inflammation vésicale.

L'utilisation des composés selon l'invention, à l'état de base, de sel d'addition à un acide, d'hydrate ou de solvat pharmaceutiquement acceptable, pour la préparation d'un médicament destiné à traiter les pathologies ci-dessus mentionnées fait partie intégrante de l'invention.

L'invention a également pour objet des médicaments qui comprennent un composé de formule (I), ou un sel d'addition à un acide, ou encore un hydrate ou un solvat pharmaceutiquement acceptable du composé de formule (I). Ces médicaments trouvent leur emploi en thérapeutique, notamment dans le traitement des pathologies ci-dessus mentionnées.

Selon un autre de ses aspects, la présente invention concerne des compositions pharmaceutiques renfermant en tant que principe actif, au moins un composé selon l'invention. Ces compositions pharmaceutiques contiennent une dose efficace d'un composé selon l'invention, ou un sel d'addition à un acide, ou un hydrate, ou un solvat pharmaceutiquement acceptable dudit composé, et éventuellement un ou plusieurs excipients pharmaceutiquement acceptables.
Lesdits excipients sont choisis selon la forme pharmaceutique et le mode d'administration souhaité, parmi les excipients habituels qui sont connus de l'homme du métier.

Dans les compositions pharmaceutiques de la présente invention pour l'administration orale, sublinguale, sous-cutanée, intramusculaire, intra-veineuse, topique, locale, intrathécale, intranasale, transdermique, pulmonaire, oculaire ou rectale, le principe actif de formule (I) ci-dessus, ou son sel d'addition à un acide, solvat ou hydrate éventuel, peut être administré sous forme unitaire d'administration, en mélange avec des excipients pharmaceutiques classiques, aux animaux et aux êtres humains pour la prophylaxie ou le traitement des troubles ou des maladies ci-dessus.

Les formes unitaires d'administration appropriées comprennent les formes par voie orale telles que les comprimés, les gélules molles ou dures, les poudres, les granules, les chewing-gums et les solutions ou suspensions orales, les formes d'administration sublinguale, buccale, intratrachéale, intraoculaire, intranasale, par inhalation, les formes d'administration sous-cutanée, intramusculaire ou intraveineuse et les formes d'administration rectale ou vaginale. Pour l'application topique, on peut utiliser les composés selon l'invention dans des crèmes, pommades ou lotions.

A titre d'exemple, une forme unitaire d'administration d'un composé selon l'invention sous forme de comprimé peut comprendre les composants suivants :

| | |
|---|---|
| Composé selon l'invention | 50,0 mg |
| Mannitol | 223,75 mg |
| Croscaramellose sodique | 6,0 mg |
| Amidon de maïs | 15,0 mg |
| Hydroxypropyl-méthylcellulose | 2,25 mg |
| Stéarate de magnésium | 3,0 mg |

Lesdites formes unitaires sont dosées pour permettre une administration journalière de 0,01 à 20 mg de principe actif par kg de poids corporel, selon la forme galénique.

Il peut y avoir des cas particuliers où des dosages plus élevés ou plus faibles sont appropriés, de tels dosages appartiennent également à l'invention. Selon la pratique habituelle, le dosage approprié à chaque patient est déterminé par le médecin selon le mode d'administration, le poids et la réponse dudit patient.
L'invention selon un autre de ses aspects, concerne également une méthode de traitement des pathologies ci-dessus indiquées qui comprend l'administration d'une dose efficace d'un composé selon l'invention, d'un de ses sels d'addition à un acide pharmaceutiquement acceptable, d'un solvat ou d'un hydrate dudit composé.

## Revendications

1. Composé répondant à la formule (I) 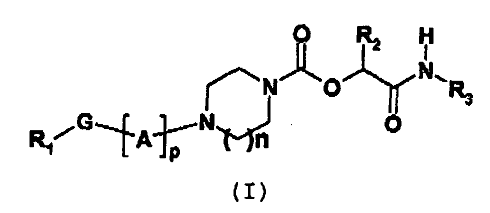 dans laquelle
n représente un nombre entier égal à 1 ou 2 ;
p représente un nombre entier allant de 1 à 7 ;
A est choisi parmi un ou plusieurs groupes X, Y et/ou Z ;
X représente un groupe méthylène éventuellement substitué par un ou deux groupes C₁₋₆-alkyle, C₃₋₇-cycloalkyle ou C₃₋₇-cycloalkyl-C₁₋₃-alkylène;
Y représente soit un groupe C₂-alcènylène éventuellement substitué par un ou deux groupes C₁₋₆-alkyle, C₃₋₇-cycloalkyle ou C₃₋₇-cycloalkyl-C₁₋₃-alkylène; soit un groupe C₂-alcynylène ;
Z représente un groupe de formule : 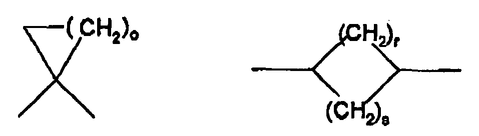
o représente un nombre entier allant de 1 à 5 ;
r et s représentent des nombres entiers et sont définis tels que r+s soit un nombre allant de 1 à 5 ;
G représente une liaison simple, un atome d'oxygène ou de soufre, un groupe SO, SO₂, C=O ou CH (OH) ;
R₁ représente un groupe R₄ éventuellement substitué par un ou plusieurs groupes R₅ et/ou R₆ ;
R₄ représente un groupe choisi parmi un phényle, pyridinyle, pyrimidinyle, pyridazinyle, pyrazinyle, triazinyle, pyrrolyle, furanyle, thiènyle, imidazolyle, oxazolyle, thiazolyle, pyrazolyle, isoxazolyle, isothiazolyle, oxadiazolyle, thiadiazolyle, triazolyle, tétrazolyle, naphtalènyle, diphénylméthyle, quinolinyle, tétrahydroquinolinyle, isoquinolinyle, tétrahydroisoquinolinyle, quinazolinyle, quinoxalinyle, phthalazinyle, cinnolinyle, naphthyridinyle, benzofuranyle, dihydrobenzofuranyle, benzothiènyle, dihydrobenzothiènyle, indolyle, indolinyle, indanyle, indazolyle, isoindolyle, benzimidazolyle, benzoxazolyle, benzisoxazolyle, benzothiazolyle, benzisothiazolyle, benzotriazolyle, benzoxadiazolyle, benzothiadiazolyle, pyrrolopyridinyle, furopyridinyle, thiènopyridinyle, imidazopyridinyle, oxazolopyridinyle, thiazolopyridinyle, pyrazolopyridinyle, isoxazolopyridinyle, isothiazolopyridinyle ;
R₅ représente un atome d'halogène ou un groupe cyano, nitro, C₁₋₆-alkyle, C₁₋₆-alcoxy, hydroxyle, C₁₋₆-thioalkyle, C₁₋₆-fluoroalkyle, C₁₋₆-fluoroalcoxy, C₁₋₆-fluorothioalkyle, NR₇R₈, NR₇COR₈, NR₇CO₂R₈, NR₇SO₂R₈, COR₇, CO₂R₇, CONR₇R₈, SO₂R₇, SO₂NR₇R₈ ou -O-(C₁₋₃-alkylène)-O ;
R₆ représente un groupe phényle, phényloxy, benzyloxy, naphtalènyle, pyridinyle, pyrimidinyle, pyridazinyle ou pyrazinyle ; le ou les groupes R₆ étant éventuellement substitués par un ou plusieurs groupes R₅ identiques ou différents l'un de l'autre;
R₇ et R₈ représentent indépendamment l'un de l'autre un atome d'hydrogène ou un groupe C₁₋₆-alkyle, ou forment avec le ou les atomes qui les portent un cycle choisi parmi azétidine, pyrrolidine, pipéridine, morpholine, thiomorpholine, azépine, pipérazine, ce cycle étant éventuellement substitué par un groupe C₁₋₆-alkyle ou benzyle ;
R₂ représente un atome d'hydrogène ou un groupe C₁₋₆-alkyle ;
R₃ représente un atome d'hydrogène ou un groupe C₁₋₆-alkyle, C₃₋₇-cycloalkyle, C₃₋₇-cycloalkyl-C₁₋₃-alkyle ;
à l'état de base, de sel d'addition à un acide, d'hydrate ou de solvat.

2. Composé de formule (I) selon la revendication 1 **caractérisé en ce que**
n représente un nombre entier égal à 1 ou 2 :
p représente un nombre entier allant de 1 à 7 ;
A est choisi parmi un ou plusieurs groupes X et/ou Y ;
X représente un groupe méthylène éventuellement substitué par un ou deux groupes C₁₋₆-alkyle;
Y représente soit un groupe C₂-alcènylène, soit un groupe C₂-alcynylène ;
G représente une liaison simple, un atome d'oxygène ou un groupe C=0 ;
R₁ représente un groupe R₄ éventuellement substitué par un ou plusieurs groupes R₅ et/ou R₆ ;
R₄ représente un groupe choisi parmi un phényle, naphtalènyle, diphénylméthyle, quinolinyle, indolyle, pyrazolyle, isoxazolyle, pyrimidinyle, thiazolyle ;
R₅ représente un atome d'halogène, ou un groupe cyano, C₁₋₆-alkyle, C₁₋₆-alcoxy, C₁₋₆-fluoroalkyle, C₁₋₆-fluoroalcoxy, ou -O-(C₁₋₃-alkylène)-O ;
R₆ représente un groupe phényle, naphtalènyle ou benzyloxy ;
R₂ représente un atome d'hydrogène ou un groupe C₁₋₆-alkyle ;
R₃ représente un atome d'hydrogène ou un groupe C₁₋₆-alkyle, C₃₋₇-cycloalkyle, C₃₋₇-cycloalkyl-C₁₋₃-alkyle;
à l'état de base, de sel d'addition à un acide, d'hydrate ou de solvat.

3. Composé de formule (I) selon la revendication 1 ou 2 **caractérisé en ce que**
n représente un nombre entier égal à 1 ;
p représente un nombre entier allant de 1 à 4 :
A est choisi parmi un ou plusieurs groupes X et/ou Y :
X représente un groupe méthylène éventuellement substitué par un ou deux groupes C₁₋₆-alkyle;
Y représente un groupe C₂-alcynylène :
G représente une liaison simple ou un atome d'oxygène ;
R₁ représente un groupe R₄ éventuellement substitué par un ou plusieurs groupes R₅ et/ou R₆ ;
R₄ représente un groupe choisi parmi un phényle, naphtalènyle, isoxazolyle ;
R₅ représente un atome d'halogène ou un groupe cyano, C₁₋₆-alcoxy, C₁₋₆-fluoroalkyle ;
R₆ représente un groupe phényle :
R₂ représente un atome d'hydrogène ou un groupe C₁₋₆-alkyle ;
R₃ représente un atome d'hydrogène ou un groupe C₁₋₆-alkyle, C₃₋₇-cycloalkyle, C₃₋₇-cycloalkyl-C₁₋₃-alkyle :
à l'état de base, de sel d'addition à un acide, d'hydrate ou de solvat.

4. Composé de formule (I) selon l'une quelconque des revendications 1 à 3 **caractérisé en ce que**
R₂ représente un atome d'hydrogène ;
R₃ représente un atome d'hydrogène ou un groupe C₁₋₆-alkyle, C₃₋₇-cycloalkyle ou C₃₋₇-cycloalkyl-C₁₋₃-alkyle.

5. Composé de formule (I) selon la revendication 1 choisi parmi :
- 4-(2-biphényl-3-yléthyl)pipérazine-1-carboxylate de 2-(méthylamino)-2-oxoéthyle .
- 4-(2-biphényl-4-yléthyl)pipérazine-1-carboxylate de 2-(méthylamino)-2-oxoéthyle
- 4-[2-(1-naphtyl)éthyl]pipérazine-1-carboxylate de 2-(méthylamino)-2-oxoéthyle
- 4-{2-[3-(4-chlorophényl)isoxazol-5-yl]éthyl}pipérazine-1-carboxylate de 2-(méthylamino)-2-oxoéthyle
- 4-{2-[5-(4-chlorophényl)isoxazol-3-yl]éthyl}pipérazine-1-carboxylate de 2-(méthylamino)-2-oxoéthyle
- 4-(3-biphényl-3-ylpropyl)pipérazine-1-carboxylate de 2-(méthylamino)-2-oxoéthyle
- 4-(3-biphényl-4-ylpropyl)pipérazine-1-carboxylate de 2-(méthylamino)-2-oxoéthyle
- 4-(3-biphényl-3-yl-1,1-diméthylpropyl)pipérazine-1-carboxylate de 2-(méthylamino)-2-oxoéthyle
- 4-[3-(3'-chlorobiphényl-3-yl)propyl]pipérazine-1-carboxylate de 2-(méthylamino)-2-oxoéthyle
- 4-[3-(4'-chlorobiphényl-3-yl)propyl]pipérazine-1-carboxylate de 2-(méthylamino)-2-oxoéthyle
- 4-[3-(3'-methoxybiphényl-3-yl)propyl]pipérazine-1-carboxylate de 2-(méthylamino)-2-oxoéthyle
- 4-[3-(4'-methoxybiphényl-3-yl)propyl]pipérazine-1-carboxylate de 2-(méthylamino)-2-oxoéthyle
- 4-[3-(3'-chlorobiphényl-4-yl)propyl]pipérazine-1-carboxylate de 2-(méthylamino)-2-oxoéthyle
- 4-[3-(4'-chlorobiphényl-4-yl)propyl]pipérazine-1-carboxylate de 2-(méthylamino)-2-oxoéthyle
- 4-[3-(2-naphtyl)propyl]pipérazi.ne-1-carboxylate de 2-(méthylamino)-2-oxoéthyle
- 4-{3-[5-(4-chlorophényl)isoxazol-3-yl)propyl]pipérazine-1-carboxylate de 2-(méthylamino)-2-oxoéthyle
- 4-{3-[3-(4-chlorophényl)isoxazol-5-yl]propyl}pipérazine-1-carboxylate de 2-(méthylamino)-2-oxoéthyle
- 4-[4-(3-chlorophényl)butyl]pipérazine-1-carboxylate de 2-(méthylamino)-2-oxoéthyle
- 4-[4-(4-chlorophényl)butyl]pipérazine-1-carboxylate de 2-(méthylamino)-2-oxoéthyle
- 4-{4-[3-(trifluorométhyl)phényl]butyl}pipérazine-1-carboxylate de 2-(méthylamino)-2-oxoéthyle
- 4-{4-[4-(trifluorométhyl)phényl]butyl}pipérazine-1-carboxylate de 2-(méthylamino)-2-oxoéthyle
- 4-{4-[4-(trifluorométhyl)phényl]but-3-yn-1-yl}pipérazine-1-carboxylate de 2-(méthylamino)-2-oxoéthyle
- 4-[5-(3-chlorophényl)pent-4-yn-1-yl]pipérazine-1-carboxylate de 2-(méthylamino)-2-oxoéthyle
- 4-[5-(2,4-dichlorophényl)pent-4-yn-1-yl]pipérazine-1-carboxylate de 2-(méthylamino)-2-oxoéthyle
- 4-[5-(2,5-dichlorophényl)pent-4-yn-1-yl]pipérazine-1-carboxylate de 2-(méthylamino)-2-oxoéthyle
- 4-[5-(3,4-dichlorophényl)pent-4-yn-1-yl]pipérazine-1-carboxylate de 2-(méthylamino)-2-oxoéthyle
- 4-[5-(3-chloro-4-fluorophényl)pent-4-yn-1-yl]pipérazine-1-carboxylate de 2-(méthylamino)-2-oxoéthyle
- 4-[3-(2-chlorophénoxy)propyl]pipérazine-1-carboxylate de 2-(méthylamino)-2-oxoéthyle
- 4-[3-(3-chlorophénoxy)propyl]pipérazine-1-carboxylate de 2-(méthylamino)-2-oxoéthyle
- 4-[3-(4-chlorophénoxy)propyl]pipérazine-1-carboxylate de 2-(méthylamino)-2-oxoéthyle
- 4-[3-(2,3-dichlorophénoxy)propyl]pipérazine-1-carboxylate de 2-(méthylamino)-2-oxoéthyle
- 4-[3-(2,4-dichlorophénoxy)propyl]pipérazine-1-carboxylate de 2-(méthylamino)-2-oxoéthyle
- 4-[3-(2,5-dichlorophénoxy)propyl]pipérazine-1-carboxylate de 2-(méthylamino)-2-oxoéthyle
- 4-[3-(2,6-dichlorophénoxy)propyl]pipérazine-1-carboxylate de 2-(méthylamino)-2-oxoéthyle
- 4-[3-(3,5-dichlorophénoxy)propyl]pipérazine-1-carboxylate de 2-(méthylamino)-2-oxoéthyle.

6. Procédé de préparation d'un composé de formule (I) selon l'une quelconque des revendications 1 à 5, comprenant l'étape consistant à transformer le carbamate-ester de formule générale (II) 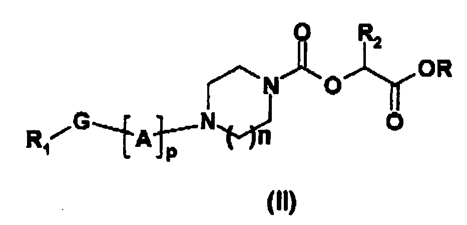 dans laquelle R₁, R₂, G, A, p et n sont tels que définis dans la formule générale (I) selon la revendication 1 et R représente un groupe méthyle ou éthyle,
par aminolyse au moyen d'une amine de formule générale R₃NH₂ dans laquelle R₃ est tel que défini dans la formule générale (I).

7. Procédé de préparation d'un composé de formule (I) selon l'une quelconque des revendications 1 à 5, comprenant l'étape consistant à transformer le carbamate-amide de formule générale (V) 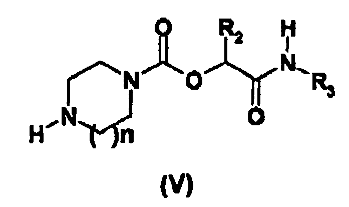 dans laquelle R₂, R₃ et n sont tels que définis dans la formule générale (I) selon la revendication 1,
par réaction avec un dérivé de formule générale R₁-G-[A]ₚ-W (VI) dans laquelle R₁, G, p et A sont tels que définis dans la formule générale (I) et W représente un atome de chlore, brome ou iode, ou un groupe mésylate ou tosylate.

8. Composé répondant à la formule générale (II), 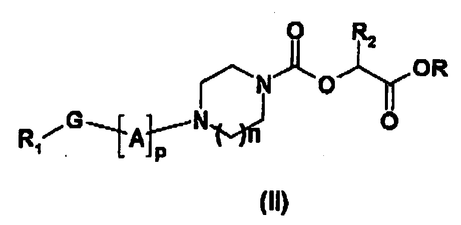 dans laquelle R₁, R₂, G, A, p et n sont tels que définis dans la formule générale (I) selon la revendication 1 et R représente un groupe méthyle ou éthyle.

9. Composé répondant à la formule générale (V), 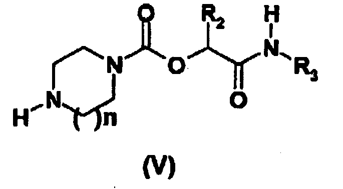 dans laquelle R₂, R₃ et n sont tels que définis dans la formule générale (I) selon la revendication 1.

10. Composé de formule (I) selon l'une quelconque des revendications 1 à 5, à l'état de base, de sel d'addition à un acide, d'hydrate ou de solvat pharmaceutiquement acceptable, pour son utilisation comme médicament.

11. Composition pharmaceutique contenant au moins un composé de formule (I) selon l'une quelconque des revendications 1 à 5, à l'état de base, de sel d'addition à un acide, d'hydrate ou de solvat pharmaceutiquement acceptable et éventuellement un ou plusieurs excipients pharmaceutiquement acceptables.

12. Utilisation d'un composé de formule (I) selon l'une quelconque des revendications 1 à 5, à l'état de base, de sel d'addition à un acide, d'hydrate ou de solvat pharmaceutiquement acceptable, pour la préparation d'un médicament destiné à prévenir ou à traiter une pathologie dans laquelle les cannabinoïdes endogènes et/ou tous autres substrats métabolisés par l'enzyme FAAH, sont impliqués.

13. Utilisation d'un composé de formule (I) selon l'une quelconque des revendications 1 à 5, à l'état de base, de sel, d'hydrate ou de solvat pharmaceutiquement acceptable, pour la préparation d'un médicament destiné à prévenir ou à traiter les douleurs aiguës ou chroniques, les vertiges, les vomissements, les nausées, les troubles du comportement alimentaire, les pathologies neurologiques et psychiatriques, les maladies neuro-dégénératives aiguës ou chroniques, l'épilepsie, les troubles du sommeil, les maladies cardiovasculaires, l'ischémie rénale, les cancers, les désordres du système immunitaire, les maladies allergiques, les maladies infectieuses parasitaires, virales ou bactériennes, les maladies inflammatoires, l'ostéoporose, les affections oculaires, les affections pulmonaires, les maladies gastro-intestinales ou l'incontinence urinaire.

## Claims

1. Compound of the formula (I) 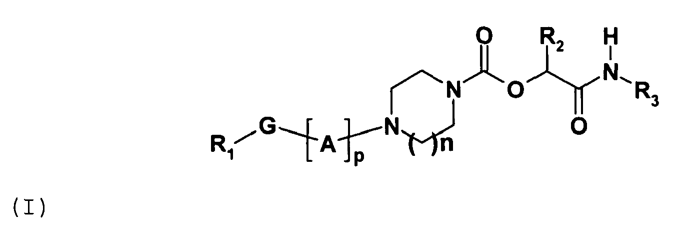 in which
n represents an integer 1 or 2;
p represents an integer ranging from 1 to 7;
A is selected from one or more groups X, Y and/or Z;
X represents a methylene group optionally substituted by one or two C₁₋₆-alkyl, C₃₋₇-cycloalkyl or C₃₋₇-cycloalkyl-C₁₋₃-alkylene groups;
Y represents either a C₂-alkenylene group optionally substituted by one or two C₁₋₆-alkyl, C₃₋₇-cycloalkyl or C₃₋₇-cycloalkyl-C₁₋₃-alkylene groups; or a C₂-alkynylene group;
Z represents a group of formula: 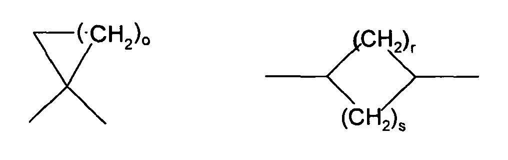
o represents an integer ranging from 1 to 5;
r and s represent integers and are defined such that r+s is a number ranging from 1 to 5;
G represents a single bond, an oxygen or sulphur atom or an SO, SO₂, C=O or CH (OH) group;
R₁ represents a group R₄ optionally substituted by one or more groups R₅ and/or R₆;
R₄ represents a group selected from a phenyl, pyridinyl, pyrimidinyl, pyridazinyl, pyrazinyl, triazinyl, pyrrolyl, furanyl, thienyl, imidazolyl, oxazolyl, thiazolyl, pyrazolyl, isoxazolyl, isothiazolyl, oxadiazolyl, thiadiazolyl, triazolyl, tetrazolyl, naphthalenyl, diphenylmethyl, quinolinyl, tetrahydroquinolinyl, isoquinolinyl, tetrahydroisoquinolinyl, quinazolinyl, quinoxalinyl, phthalazinyl, cinnolinyl, naphthyridinyl, benzofuranyl, dihydrobenzofuranyl, benzothienyl, dihydrobenzothienyl, indolyl, indolinyl, indanyl, indazolyl, isoindolyl, benzimidazolyl, benzoxazolyl, benzisoxazolyl, benzothiazolyl, benzisothiazolyl, benzotriazolyl, benzoxadiazolyl, benzothiadiazolyl, pyrrolopyridinyl, furopyridinyl, thienopyridinyl, imidazopyridinyl, oxazolopyridinyl, thiazolopyridinyl, pyrazolopyridinyl, isoxazolopyridinyl and isothiazolopyridinyl;
R₅ represents a halogen atom or a cyano, nitro, C₁₋₆-alkyl, C₁₋₆-alkoxy, hydroxyl, C₁₋₆-thioalkyl, C₁₋₆-fluoroalkyl, C₁₋₆-fluoroalkoxy or C₁-C₆-fluorothioalkyl group, a group NR₇R₈, NR₇COR₈, NR₇CO₂R₈, NR₇SO₂R₈, COR₇, CO₂R₇, CONR₇R₈, SO₂R₇ or SO₂NR₇R₈, or an -O-(C₁₋₃-alkylene)-O group;
R₆ represents a phenyl, phenyloxy, benzyloxy, naphthalenyl, pyridinyl, pyrimidinyl, pyridazinyl or pyrazinyl group, the group or groups R₆ being optionally substituted by one or more groups R₅ identical to or different from one another;
R₇ and R₈ represent independently of one another a hydrogen atom or a C₁₋₆-alkyl group, or form with the atom or atoms which carry them a ring selected from azetidine, pyrrolidine, piperidine, morpholine, thiomorpholine, azepine and piperazine, this ring being optionally substituted by a C₁₋₆-alkyl or benzyl group;
R₂ represents a hydrogen atom or a C₁₋₆-alkyl group;
R₃ represents a hydrogen atom or a C₁₋₆-alkyl, C₃₋₇-cycloalkyl or C₃₋₇-cycloalkyl-C₁₋₃alkyl group;
in the form of a base, addition salt with an acid, hydrate or solvate.

2. Compound of formula (I) according to Claim 1, **characterized in that**
n represents an integer 1 or 2;
p represents an integer ranging from 1 to 7;
A is selected from one or more groups X and/or Y;
X represents a methylene group optionally substituted by one or two C₁₋₆-alkyl groups;
Y represents either a C₂-alkenylene group or a C₂-alkynylene group;
G represents a single bond, an oxygen atom or a C=O group;
R₁ represents a group R₄ optionally substituted by one or more groups R₅ and/or R₆;
R₄ represents a group selected from a phenyl, naphthalenyl, diphenylmethyl, quinolinyl, indolyl, pyrazolyl, isoxazolyl, pyrimidinyl and thiazolyl;
R₅ represents a halogen atom or a cyano group, a C₁₋₆-alkyl group, a C₁₋₆-alkoxy group, a C₁₋₆-fluoroalkyl group, a C₁-C₆-fluoroalkoxy group, or an -O-(C₁₋₃-alkylene)-O group;
R₆ represents a phenyl, naphthalenyl or benzyloxy group;
R₂ represents a hydrogen atom or a C₁₋₆-alkyl group;
R₃ represents a hydrogen atom or a C₁₋₆-alkyl, C₃₋₇-cycloalkyl or C₃₋₇-cycloalkyl-C₁₋₃-alkyl group;
in the form of a base, addition salt with an acid, hydrate or solvate.

3. Compound of formula (I) according to Claim 1 or 2, **characterized in that**
n represents the integer 1;
p represents an integer ranging from 1 to 4;
A is selected from one or more groups X and/or Y;
X represents a methylene group optionally substituted by one or two C₁₋₆-alkyl groups;
Y represents a C₂-alkynylene group;
G represents a single bond or an oxygen atom;
R₁ represents a group R₄ optionally substituted by one or more groups R₅ and/or R₆;
R₄ represents a group selected from a phenyl, naphthalenyl or isoxazolyl;
R₅ represents a halogen atom or a cyano group, a C₁₋₆-alkoxy group, a C₁₋₆-fluoroalkyl group;
R₆ represents a phenyl group;
R₂ represents a hydrogen atom or C₁₋₆-alkyl group;
R₃ represents a hydrogen atom or a C₁₋₆-alkyl, C₃₋₇-cycloalkyl or C₃₋₇-cycloalkyl-C₁₋₃-alkyl group;
in the form of a base, addition salt with an acid, hydrate or solvate.

4. Compound of formula (I) according to any one of Claims 1 to 3, **characterized in that**
R₂ represents a hydrogen atom;
R₃ represents a hydrogen atom or a C₁₋₆-alkyl, C₃₋₇-cycloalkyl or C₃₋₇-cycloalkyl-C₁₋₃-alkyl group.

5. Compound of formula (I) according to Claim 1, selected from:
- 2-(methylamino)-2-oxoethyl 4-(2-biphenyl-3-ylethyl)-piperazine-1-carboxylate
- 2-(methylamino)-2-oxoethyl 4-(2-biphenyl-4-ylethyl)-piperazine-1-carboxylate
- 2-(methylamino)-2-oxoethyl 4-[2-(1-naphthyl)ethyl]-piperazine-1-carboxylate
- 2-(methylamino)-2-oxoethyl 4-{2-[3-(4-chlorophenyl)-isoxazol-5-yl]ethyl}piperazine-1-carboxylate
- 2-(methylamino)-2-oxoethyl 4-{2-[5-(4-chlorophenyl)-isoxazol-3-yl]ethyl}piperazine-1-carboxylate
- 2-(methylamino)-2-oxoethyl 4-(3-biphenyl-3-ylpropyl)-piperazine-1-carboxylate
- 2-(methylamino)-2-oxoethyl 4-(3-biphenyl-4-ylpropyl)-piperazine-1-carboxylate
- 2-(methylamino)-2-oxoethyl 4-(3-biphenyl-3-yl-1,1-dimethylpropyl)piperazine-1-carboxylate
- 2-(methylamino)-2-oxoethyl 4-[3-(3'-chlorobiphenyl-3-yl)propyl]piperazine-1-carboxylate
- 2-(methylamino)-2-oxoethyl 4-[3-(4'-chlorobiphenyl-3-yl)propyl]piperazine-1-carboxylate
- 2-(methylamino)-2-oxoethyl 4-[3-(3'-methoxybiphenyl-3-yl)propyl]piperazine-1-carboxylate
- 2-(methylamino)-2-oxoethyl 4-[3-(4'-methoxybiphenyl-3-yl)propyl]piperazine-1-carboxylate
- 2-(methylamino)-2-oxoethyl 4-[3-(3'-chlorobiphenyl-4-yl)propyl]piperazine-1-carboxylate
- 2-(methylamino)-2-oxoethyl 4-[3-(4'-chlorobiphenyl-4-yl)propyl]piperazine-1-carboxylate
- 2-(methylamino)-2-oxoethyl 4-[3-(2-naphthyl)propyl]-piperazine-1-carboxylate
- 2-(methylamino)-2-oxoethyl 4-{3-[5-(4-chlorophenyl)-isoxazol-3-yl]propyl}piperazine-1-carboxylate
- 2-(methylamino)-2-oxoethyl 4-{3-[3-(4-chlorophenyl)-isoxazol-5-yl]propyl}piperazine-1-carboxylate
- 2-(methylamino)-2-oxoethyl 4-[4-(3-chlorophenyl)-butyl]piperazine-1-carboxylate
- 2-(methylamino)-2-oxoethyl 4-[4-(4-chlorophenyl)-butyl]piperazine-1-carboxylate
- 2-(methylamino)-2-oxoethyl 4-{4-[3-(trifluoromethyl)-phenyl]butyl}piperazine-1-carboxylate
- 2-(methylamino)-2-oxoethyl 4-{4-[4-(trifluoromethyl)-phenyl]butyl}piperazine-1-carboxylate
- 2-(methylamino)-2-oxoethyl 4-{4-[4-(trifluoromethylphenyl]but-3-yn-1-yl}piperazine-1-carboxylate
- 2-(methylamino)-2-oxoethyl 4-[5-(3-chlorophenyl)pent-4-yn-1-yl]piperazine-1-carboxylate
- 2-(methylamino)-2-oxoethyl 4-[5-(2,4-dichlorophenyl)-pent-4-yn-1-yl]piperazine-1-carboxylate
- 2-(methylamino)-2-oxoethyl 4-[5-(2,5-dichlorophenyl)-pent-4-yn-1-yl]piperazine-1-carboxylate
- 2-(methylamino)-2-oxoethyl 4-[5-(3,4-dichlorophenyl)pent-4-yn-1-yl]piperazine-1-carboxylate
- 2-(methylamino)-2-oxoethyl 4-[5-(3-chloro-4-fluorophenyl)pent-4-yn-1-yl]piperazine-1-carboxylate
- 2-(methylamino)-2-oxoethyl 4-[3-(2-chlorophenoxy)propyl]piperazine-1-carboxylate
- 2-(methylamino)-2-oxoethyl 4-[3-(3-chlorophenoxy)-propyl]piperazine-1-carboxylate
- 2-(methylamino)-2-oxoethyl 4-[3-(4-chlorophenoxy)-propyl]piperazine-1-carboxylate
- 2-(methylamino)-2-oxoethyl 4-[3-(2,3-dichlorophenoxy)propyl]piperazine-1-carboxylate
- 2-(methylamino)-2-oxoethyl 4-[3-(2,4-dichlorophenoxy)propyl]piperazine-1-carboxylate
- 2-(methylamino)-2-oxoethyl 4-[3-(2,5-dichlorophenoxy)propyl]piperazine-1-carboxylate
- 2-(methylamino)-2-oxoethyl 4-[3-(2,6-dichlorophenoxy)propyl]piperazine-1-carboxylate
- 2-(methylamino)-2-oxoethyl 4-[3-(3,5-dichlorophenoxy)propyl]piperazine-1-carboxylate.

6. Process for preparing a compound of formula (I) according to any one of Claims 1 to 5, comprising the step consisting in converting the carbamate ester of general formula (II) 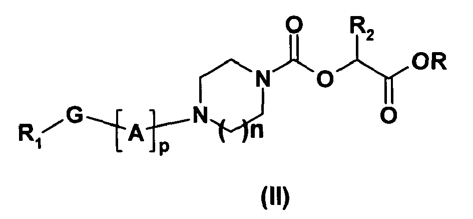 in which R₁, R₂, G, A, p and n are as defined in the general formula (I) according to Claim 1 and R represents a methyl or ethyl group,
by aminolysis using an amine of general formula R₃NH₂ in which R₃ is as defined in the general formula (I).

7. Process for preparing a compound of formula (I) according to any one of Claims 1 to 5, comprising the step consisting in converting the carbamate-amide of general formula (V) 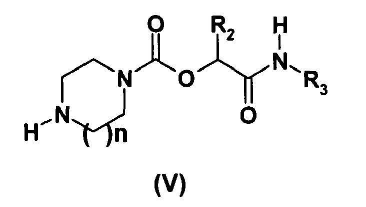 in which R₂, R₃ and n are as defined in the general formula (I) according to Claim 1,
by reaction with a derivative of general formula R₁-G-[A]ₚ-W (VI), in which R₁, G, p and A are as defined in the general formula (I) and W represents a chlorine, bromine or iodine atom, or a mesylate or tosylate group.

8. Compound of the general formula (II), 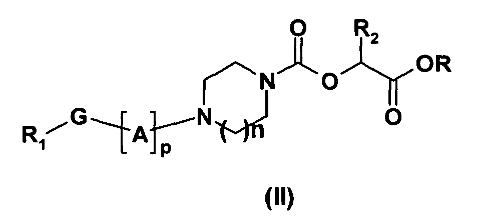 in which R₁, R₂, G, A, p and n are as defined in the general formula (I) according to Claim 1 and R represents a methyl or ethyl group.

9. Compound of the general formula (V), 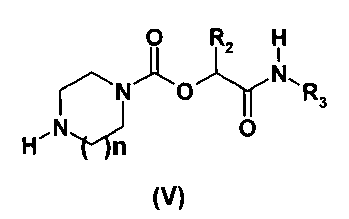 in which R₂, R₃ and n are as defined in the general formula (I) according to Claim 1.

10. Compound of formula (I) according to any one of Claims 1 to 5, in the form of a base, addition salt with an acid, hydrate or pharmaceutically acceptable solvate, for its use as a medicinal product.

11. Pharmaceutical composition comprising at least one compound of formula (I) according to any one of Claims 1 to 5, in the form of a base, addition salt with an acid, hydrate or pharmaceutically acceptable solvate, and optionally one or more pharmaceutically acceptable excipients.

12. Use of a compound of formula (I) according to any one of Claims 1 to 5, in the form of a base, addition salt with an acid, hydrate or pharmaceutically acceptable solvate, for preparing a medicinal product intended for preventing or treating a pathology in which endogenous cannabinoids and/or any other substrates metabolized by the enzyme FAAH are involved.

13. Use of a compound of formula (I) according to any one of Claims 1 to 5, in the form of a base, salt, hydrate or pharmaceutically acceptable solvate, for preparing a medicinal product intended for preventing or treating acute or chronic pain, dizziness, vomiting, nausea, eating disorders, neurological and psychiatric pathologies, acute or chronic neurodegenerative diseases, epilepsy, sleep disorders, cardiovascular diseases, renal ischemia, cancers, disorders of the immune system, allergic diseases, parasitic, viral or bacterial infectious diseases, inflammatory diseases, osteoporosis, ocular conditions, pulmonary conditions, gastrointestinal diseases or urinary incontinence.

## Patentansprüche

1. Verbindung der Formel (I) 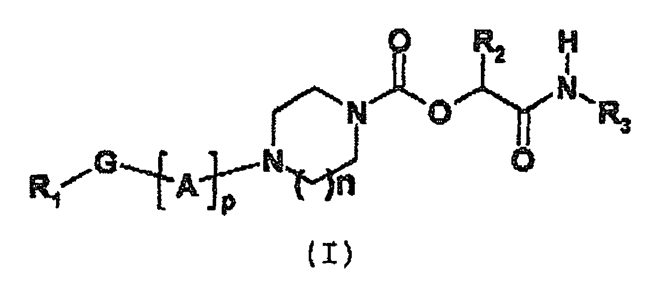 worin
n für eine ganze Zahl mit einem Wert von 1 oder 2 steht;
p für eine ganze Zahl von 1 bis 7 steht;
A unter einer oder mehreren Gruppen X, Y und/oder Z ausgewählt ist;
X für eine gegebenenfalls durch eine oder zwei C₁₋₆-Alkyl-, C₃₋₇-Cycloalkyl- oder C₃₋₇-Cycloalkyl-C₁₋₃-alkylengruppen substituierte Methylengruppe steht;
Y entweder für eine gegebenenfalls durch eine oder zwei C₁₋₆-Alkyl-, C₃₋₇-Cycloalkyl- oder C₃₋₇-Cycloalkyl-C₁₋₃-alkylengruppen substituierte C₂-Alkenylengruppe oder eine C₂-Alkinylengruppe steht; Z für eine Gruppe der Formel: 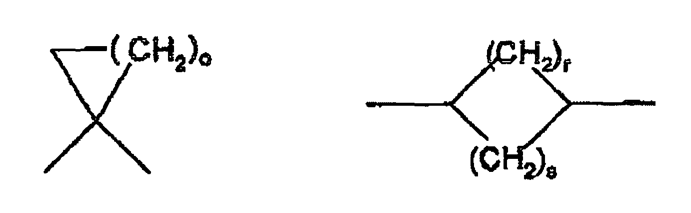 steht;
o für eine ganze Zahl von 1 bis 5 steht;
r und s für ganze Zahlen stehen und so definiert sind, daß r+s für eine ganze Zahl von 1 bis 5 steht;
G für eine Einfachbindung, ein Sauerstoff- oder Schwefelatom oder eine SO-, SO₂- C=O- oder CH(OH)-Gruppe steht;
R₁ für eine gegebenenfalls durch eine oder mehrere Gruppen R₅ und/oder R₆ substituierte Gruppe R₄ steht;
R₄ für eine unter Phenyl, Pyridinyl, Pyrimidinyl, Pyridazinyl, Pyrazinyl, Triazinyl, Pyrrolyl, Furanyl, Thienyl, Imidazolyl, Oxazolyl, Thiazolyl, Pyrazolyl, Isoxazolyl, Isothiazolyl, Oxadiazolyl, Thiadiazolyl, Triazolyl, Tetrazolyl, Naphthalinyl, Diphenylmethyl, Chinolinyl, Tetrahydrochinolinyl, Isochinolinyl, Tetrahydroisochinolinyl, Chinazolinyl, Chinoxalinyl, Phthalazinyl, Cinnolinyl, Naphthyridinyl, Benzofuranyl, Dihydrobenzofuranyl, Benzothienyl, Dihydrobenzothienyl, Indolyl, Indolinyl, Indanyl, Indazolyl, Isoindolyl, Benzimidazolyl, Benzoxazolyl, Benzisoxazolyl, Benzothiazolyl, Benzisothiazolyl, Benzotriazolyl, Benzoxadiazolyl, Benzothiadiazolyl, Pyrrolopyridinyl, Furopyridinyl, Thienopyridinyl, Imidazopyridinyl, Oxazolopyridinyl, Thiazolopyridinyl, Pyrazolopyridinyl, Isoxazolopyridinyl und Isothiazolopyridinyl ausgewählte Gruppe steht;
R₅ für ein Halogenatom oder eine Cyano-, Nitro-, C₁₋₆-Alkyl-, C₁₋₆-Alkoxy-, Hydroxyl-, C₁₋₆-Thioalkyl-, C₁₋₆-Fluoralkyl-, C₁₋₆-Fluoralkoxy-, C₁-C₆-Fluorthioalkyl-, NR₇R₈-, NR₇COR₈-, NR₇CO₂R₈-, NR₇SO₂R₈-, COR₇-, CO₂R₇-, CONR₇R₈-, SO₂R₇-, SO₂NR₇R₈- oder -O-(C₁₋₃-Alkylen)-O-Gruppe steht;
R₆ für eine Phenyl-, Phenyloxy-, Benzyloxy-, Naphthalinyl-, Pyridinyl-, Pyrimidinyl-, Pyridazinyl- oder Pyrazinylgruppe steht; wobei die Gruppe bzw. Gruppen R₆ gegebenenfalls durch einen oder mehrere gleiche oder voneinander verschiedene Gruppen R₅ substituiert sein kann bzw. können;
R₇ und R₈ unabhängig voneinander für ein Wasserstoffatom oder eine C₁₋₆-Alkylgruppe stehen oder gemeinsam mit dem Atom bzw. den Atomen, an das bzw. die sie gebunden sind, einen unter Azetidin, Pyrrolidin, Piperidin, Morpholin, Thiomorpholin, Azepin und Piperazin ausgewählten Ring bilden, welcher gegebenenfalls durch eine C₁₋₆-Alkyl- oder Benzylgruppe substituiert ist;
R₂ für ein Wasserstoffatom oder eine C₁₋₆-Alkylgruppe steht;
R₃ für ein Wasserstoffatom oder eine C₁₋₆-Alkyl-, C₃₋₇-Cycloalkyl- oder C₃₋₇-Cycloalkyl-C₁₋₃-alkylgruppe steht;
in Basen-, Säureadditionssalz-, Hydrat- oder Solvatform.

2. Verbindung der Formel (I) nach Anspruch 1, **dadurch gekennzeichnet, daß**
n für eine ganze Zahl mit einem Wert von 1 oder 2 steht;
p für eine ganze Zahl von 1 bis 7 steht;
A unter einer oder mehreren Gruppen X und/oder Y ausgewählt ist;
X für eine gegebenenfalls durch eine oder zwei C₁₋₆-Alkylgruppen substituierte Methylengruppe steht;
Y entweder für eine C₂-Alkenylengruppe oder eine C₂-Alkinylengruppe steht;
G für eine Einfachbindung, ein Sauerstoffatom oder eine C=O-Gruppe steht;
R₁ für eine gegebenenfalls durch eine oder mehrere Gruppen R₅ und/oder R₆ substituierte Gruppe R₄ steht;
R₄ für eine unter Phenyl, Naphthalinyl, Diphenylmethyl, Chinolinyl, Indolyl, Pyrazolyl, Isoxazolyl, Pyrimidinyl und Thiazolyl ausgewählte Gruppe steht;
R₅ für ein Halogenatom oder eine Cyano-, C₁₋₆-Alkyl-, C₁₋₆-Alkoxy-, C₁₋₆-Fluoralkyl-, C₁₋₆-Fluoralkoxy- oder -O-(C₁₋₃-Alkylen)-O-Gruppe steht;
R₆ für eine Phenyl-, Naphthalinyl-, oder Benzyloxygruppe steht;
R₂ für ein Wasserstoffatom oder eine C₁₋₆-Alkylgruppe steht;
R₃ für ein Wasserstoffatom oder eine C₁₋₆-Alkyl-, C₃₋₇-Cycloalkyl- oder C₃₋₇-Cycloalkyl-C₁₋₃-alkylgruppe steht;
in Basen-, Säureadditionssalz-, Hydrat- oder Solvatform.

3. Verbindung der Formel (I) nach Anspruch 1 oder 2, **dadurch gekennzeichnet, daß**
n für eine ganze Zahl mit einem Wert von 1 steht;
p für eine ganze Zahl von 1 bis 4 steht;
A unter einer oder mehreren Gruppen X und/oder Y ausgewählt ist;
X für eine gegebenenfalls durch eine oder zwei C₁₋₆-Alkylgruppen substituierte Methylengruppe steht;
Y entweder für eine C₂-Alkinylengruppe steht;
G für eine Einfachbindung oder ein Sauerstoffatom steht;
R₁ für eine gegebenenfalls durch eine oder mehrere Gruppen R₅ und/oder R₆ substituierte Gruppe R₄ steht;
R₄ für eine unter Phenyl, Naphthalinyl und Isoxazolyl ausgewählte Gruppe steht;
R₅ für ein Halogenatom oder eine Cyano-, C₁₋₆-Alkoxy- oder C₁₋₆-Fluoralkylgruppe steht;
R₆ für eine Phenylgruppe steht;
R₂ für ein Wasserstoffatom oder eine C₁₋₆-Alkylgruppe steht;
R₃ für ein Wasserstoffatom oder eine C₁₋₆-Alkyl-, C₃₋₇-Cycloalkyl- oder C₃₋₇-Cycloalkyl-C₁₋₃-alkylgruppe steht;
in Basen-, Säureadditionssalz-, Hydrat- oder Solvatform.

4. Verbindung der Formel (I) nach einem der Ansprüche 1 bis 3, **dadurch gekennzeichnet, daß**
R₂ für ein Wasserstoffatom steht;
R₃ für ein Wasserstoffatom oder eine C₁₋₆-Alkyl-, C₃₋₇-Cycloalkyl- oder C₃₋₇-Cycloalkyl-C₁₋₃-alkylgruppe steht;

5. Verbindung der Formel (I) nach Anspruch 1, ausgewählt unter:
- 4-(2-Biphenyl-3-ylethyl)piperazin-1-carbonsäure-2-(methylamino)-2-oxoethylester
- 4-(2-Biphenyl-4-ylethyl)piperazin-1-carbonsäure-2-(methylamino)-2-oxoethylester
- 4-[2-(1-Naphthyl)ethyl]piperazin-1-carbonsäure-2-(methylamino)-2-oxoethylester
- 4-{2-[3-(4-Chlorphenyl)isoxazol-5-yl]ethyl}-piperazin-1-carbonsäure-2-(methylamino)-2-oxoethylester
- 4-{2-[5-(4-Chlorphenyl)isoxazol-3-yl]ethyl}-piperazin-1-carbonsäure-2-(methylamino)-2-oxoethylester
- 4-(3-Biphenyl-3-ylpropyl)piperazin-1-carbonsäure-2-(methylamino)-2-oxoethylester
- 4-(3-Biphenyl-4-ylpropyl)piperazin-1-carbonsäure-2-(methylamino)-2-oxoethylester
- 4-(3-Biphenyl-3-yl-1,1-dimethylpropyl)piperazin-1-carbonsäure-2-(methylamino)-2-oxoethylester
- 4-[3-(3'-Chlorbiphenyl-3-yl)propyl]piperazin-1-carbonsäure-2-(methylamino)-2-oxoethylester
- 4-[3-(4'-Chlorbiphenyl-3-yl)propyl]piperazin-1-carbonsäure-2-(methylamino)-2-oxoethylester
- 4-[3-(3'-Methoxybiphenyl-3-yl)propyl]piperazin-1-carbonsäure-2-(methylamino)-2-oxoethylester
- 4-[3-(4'-Methoxybiphenyl-3-yl)propyl]piperazin-1-carbonsäure-2-(methylamino)-2-oxoethylester
- 4-[3-(3'-Chlorbiphenyl-4-yl)propyl]piperazin-1-carbonsäure-2-(methylamino)-2-oxoethylester
- 4-[3-(4'-Chlorbiphenyl-4-yl)propyl]piperazin-1-carbonsäure-2-(methylamino)-2-oxoethylester
- 4-[3-(2-Naphthyl)propyl]piperazin-1-carbonsäure-2-(methylamino)-2-oxoethylester
- 4-{3-[5-(4-Chlorphenyl)isoxazol-3-yl]propyl}-piperazin-1-carbonsäure-2-(methylamino)-2-oxoethylester
- 4-{3-[3-(4-Chlorphenyl)isoxazol-5-yl]propyl}-piperazin-1-carbonsäure-2-(methylamino)-2-oxoethylester
- 4-[4-(3-Chlorphenyl)butyl]piperazin-1-carbonsäure-2-(methylamino)-2-oxoethylester
- 4-[4-(4-Chlorphenyl)butyl]piperazin-1-carbonsäure-2-(methylamino)-2-oxoethylester
- 4-{4-[3-(Trifluormethyl)phenyl]butyl}piperazin-1-carbonsäure-2-(methylamino)-2-oxoethylester
- 4-{4-[4-(Trifluoromethyl)phenyl]butyl}piperazin-1-carbonsäure-2-(methylamino)-2-oxoethylester
- 4-{4-[4-(Trifluormethyl)phenyl]but-3-in-1-yl}-piperazin-1-carbonsäure-2-(methylamino)-2-oxoethylester
- 4-[5-(3-Chlorphenyl)pent-4-in-1-yl]piperazin-1-carbonsäure-2-(methylamino)-2-oxoethylester
- 4-[5-(2,4-Dichlorphenyl)pent-4-in-1-yl]-piperazin-1-carbonsäure-2-(methylamino)-2-oxoethylester
- 4-[5-(2,5-Dichlorphenyl)pent-4-in-1-yl]-piperazin-1-carbonsäure-2-(methylamino)-2-oxoethylester
- 4-[5-(3,4-Dichlorphenyl)pent-4-in-1-yl]-piperazin-1-carbonsäure-2-(methylamino)-2-oxoethylester
- 4-[5-(3-Chlor-4-fluorphenyl)pent-4-in-1-yl]-piperazin-1-carbonsäure-2-(methylamino)-2-oxoethylester
- 4-[3-(2-Chlorphenoxy)propyl]piperazin-1-carbonsäure-2-(methylamino)-2-oxoethylester
- 4-[3-(3-Chlorphenoxy)propyl]piperazin-1-carbonsäure-2-(methylamino)-2-oxoethylester
- 4-[3-(4-Chlorphenoxy)propyl]piperazin-1-carbonsäure-2-(methylamino)-2-oxoethylester
- 4-[3-(2,3-Dichlorphenoxy)propyl]piperazin-1-carbonsäure-2-(methylamino)-2-oxoethylester
- 4-[3-(2,4-Dichlorphenoxy)propyl]piperazin-1-carbonsäure-2-(methylamino)-2-oxoethylester
- 4-[3-(2,5-Dichlorphenoxy)propyl]piperazin-1-carbonsäure-2-(methylamino)-2-oxoethylester
- 4-[3-(2,6-Dichlorphenoxy)propyl]piperazin-1-carbonsäure-2-(methylamino)-2-oxoethylester
- 4-[3-(3,5-Dichlorphenoxy)propyl]piperazin-1-carbonsäure-2-(methylamino)-2-oxoethylester.

6. Verfahren zur Herstellung einer Verbindung der Formel (I) nach einem der Ansprüche 1 bis 5, bei dem man den Carbamatester der allgemeinen Formel (II) 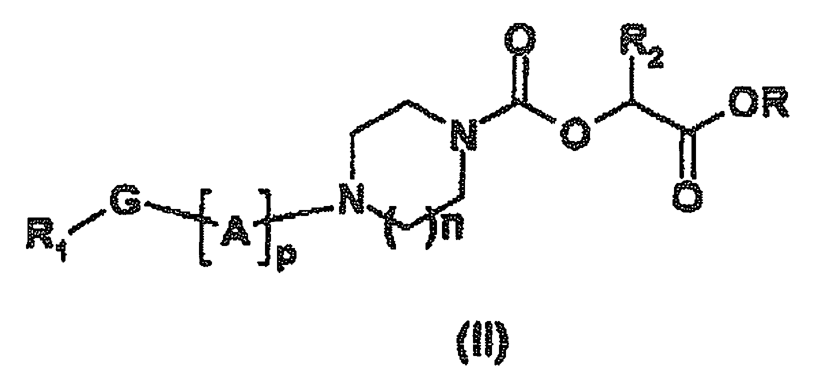 worin R₁, R₂, G, A, p und n die in der allgemeinen Formel (I) nach Anspruch 1 angegebene Bedeutung besitzen und R für eine Methyl- oder Ethylgruppe steht,
durch Aminolyse mit einem Amin der allgemeinen Formel R₃NH₂, worin R₃ die in der allgemeinen Formel (I) angegebene Bedeutung besitzt, umwandelt.

7. Verfahren zur Herstellung einer Verbindung der Formel (I) nach einem der Ansprüche 1 bis 5, bei dem man das Carbamatamid der allgemeinen Formel (V) 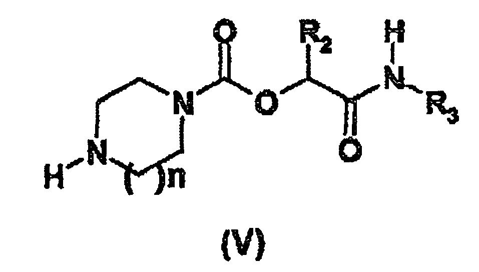 worin R₂, R₃ und n die in der allgemeinen Formel (I) nach Anspruch 1 angegebene Bedeutung besitzen, durch Umsetzung mit einem Derivat der allgemeinen Formel R₁-G-[A]ₚ-W (VI), worin R₁, G, p and A die in der allgemeinen Formel (I) angegebene Bedeutung besitzen und W für ein Chlor-, Brom- oder Iodatom oder eine Mesylat- oder Tosylatgruppe steht, umwandelt.

8. Verbindung der allgemeinen Formel (II) 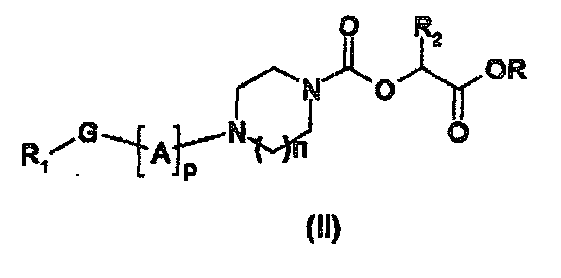 worin R₁, R₂, G, A, p und n die in der allgemeinen Formel (I) nach Anspruch 1 angegebene Bedeutung besitzen und R für eine Methyl- oder Ethylgruppe steht.

9. Verbindung der allgemeinen Formel (V) 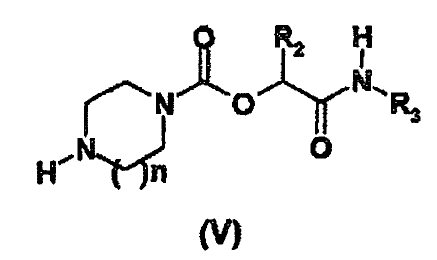 worin R₂, R₃ und n die in der allgemeinen Formel (I) nach Anspruch 1 angegebene Bedeutung besitzen.

10. Verbindung der Formel (I) nach einem der Ansprüche 1 bis 5 in pharmazeutisch unbedenklicher Basen-, Säureadditionssalz-, Hydrat- oder Solvatform zur Verwendung als Arzneimittel.

11. Pharmazeutische Zusammensetzung, enthaltend mindestens eine Verbindung der Formel (I) nach einem der Ansprüche 1 bis 5 in pharmazeutisch unbedenklicher Basen-, Säureadditionssalz-, Hydrat- oder Solvatform und gegebenenfalls einen oder mehrere pharmazeutisch unbedenkliche Trägerstoffe.

12. Verwendung einer Verbindung der Formel (I) nach einem der Ansprüche 1 bis 5 in pharmazeutisch unbedenklicher Basen-, Säureadditionssalz-, Hydrat- oder Solvatform zur Herstellung eines Arzneimittels zur Prävention oder Behandlung einer Pathologie, an der endogene Cannabinoide und/oder andere durch das Enzym FAAH metabolisierte Substanzen beteiligt sind.

13. Verwendung einer Verbindung der Formel (I) nach einem der Ansprüche 1 bis 5 in pharmazeutisch unbedenklicher Basen-, Salz-, Hydrat- oder Solvatform zur Herstellung eines Arzneimittels zur Prävention oder Behandlung von akuten oder chronischen Schmerzen, Schwindel, Erbrechen, Übelkeit, Eßstörungen, neurologischen und psychiatrischen Pathologien, akuten und chronischen neurodegenerativen Erkrankungen, Epilepsie, Schlafstörungen, Herz-Kreislauf-Erkrankungen, Nierenischämie, Krebs, Störungen des Immunsystems, allergischen Erkrankungen, parasitären, viralen oder bakteriellen Infektionserkrankungen, entzündlichen Erkrankungen, Osteoporose, Augenleiden, Lungenleiden, Magen-Darm-Erkrankungen oder Harninkontinenz.
